# EUROPÄISCHE PATENTSCHRIFT

(11) **EP 2 970 197 B1**
(45) Veröffentlichungstag und Bekanntmachung des Hinweises auf die Patenterteilung: **11.04.2018**
(21) Anmeldenummer: 14708477.6
(22) Anmeldetag: 24.02.2014
(51) Int. Cl.: C07D 401/12, C07D 403/14, C07D 409/14, C07D 417/14, C07F 1/08, C07F 1/10, C07F 1/12, C07F 3/06, C07F 5/06, C07F 19/00, C09K 11/06, H01L 51/00, H01L 51/50, C07D 401/14

(54) **KUPFER-KOMPLEXE MIT POLYPODALEN LIGANDEN, DIE ÜBER EIN GEMEINSAMES STICKSTOFF- ODER PHOSPHORATOM MITEINDANDER VERBUNDEN SIND SOWIE DEREN VERWENDUNG IN OLEDS**
COPPER COMPLEXES WITH POLYPODAL LIGANDS CONNECTED THROUGH A COMMON NITROGEN OR PHOSPHOR ATOM AS WELL AS THEIR USE IN OLEDS
COMPLEXES DE CUIVRE AVEC DES LIGANDS POLYPODAUX RELIÉS À UN AZOTE OU À UN PHOSPHORATOME COMMUNS ET LEUR UTILISATION DANS DES OLEDS

(30) Priorität: 16.03.2013 EP 13001354
(43) Veröffentlichungstag der Anmeldung: 20.01.2016
(73) Patentinhaber: Merck Patent GmbH, 64293 Darmstadt (DE)
(72) Erfinder: WESEMANN, Lars, 72072 Tübingen (DE); KLEIH, Matthias, 73770 Denkendorf (DE); MAYER, Hermann, August, 72076 Tuebingen (DE)
(86) Internationale Anmeldenummer: PCT/EP2014/000480
(87) Internationale Veröffentlichungsnummer: WO 2014/146749

(56) Entgegenhaltungen:
- WO-A1-2004/081017
- WO-A1-2011/066898
- FRY ET AL: "Intermolecular versus intramolecular electron-/atom- (Cl<?>) transfer in heme-iron and copper pyridylalkylamine complexes", INORGANICA CHIMICA ACTA, ELSEVIER BV, NL, Bd. 361, Nr. 4, 25. August 2007 (2007-08-25), Seiten 1100-1115, XP022488991, ISSN: 0020-1693, DOI: 10.1016/J.ICA.2007.08.016
- MITCHELL R. MALACHOWSKI ET AL: "Comparative study of the catalytic oxidation of catechols by copper(II) complexes of tripodal ligands", JOURNAL OF THE CHEMICAL SOCIETY, DALTON TRANSACTIONS, Nr. 1, 1995, Seite 31, XP055120124, ISSN: 0300-9246, DOI: 10.1039/dt9950000031
- BOOYONG S. LIM ET AL: "Molecular Heme-Cyanide-Copper Bridged Assemblies: Linkage Isomerism, Trends in [nu] CN Values, and Relation to the Heme- a 3 /Cu B Site in Cyanide-Inhibited Heme-Copper Oxidases", INORGANIC CHEMISTRY, Bd. 37, Nr. 19, 25. August 1998 (1998-08-25), Seiten 4898-4908, XP055120140, ISSN: 0020-1669, DOI: 10.1021/ic9801793
- DEBABRATA MAITI ET AL: "Aryl Hydroxylation from a Mononuclear Copper-Hydroperoxo Species", JOURNAL OF THE AMERICAN CHEMICAL SOCIETY, Bd. 129, Nr. 22, 5. November 2007 (2007-11-05), Seiten 6998-6999, XP055120141, ISSN: 0002-7863, DOI: 10.1021/ja071704c
- DATABASE CA [Online] CHEMICAL ABSTRACTS SERVICE, COLUMBUS, OHIO, US; 1985, KARLIN, KENNETH D. ET AL: "Synthesis and characterization of copper(II) complexes of a tetradentate N3O ligand: mononuclear analogs for phenolate-bridged dinuclear complexes", XP002725004, gefunden im STN Database accession no. 1985:515000

## Beschreibung

Die vorliegende Anmeldung betrifft Metallkomplexe, die Verwendung dieser Metallkomplexe in elektronischen Vorrichtungen sowie elektronische Vorrichtungen, insbesondere organische Elektrolumineszenzvorrichtungen, enthaltend diese Metallkomplexe.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Dabei werden als emittierende Materialien zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, die Triplettemission zeigen, immer noch Verbesserungsbedarf. So sind die physikalischen Eigenschaften von phosphoreszierenden OLEDs im Hinblick auf Effizienz, Betriebsspannung und Lebensdauer noch nicht ausreichend für die Verwendung von Triplettemittern in hochwertigen und langlebigen Elektrolumineszenzvorrichtungen. Dies gilt insbesondere für OLEDs, welche im kürzerwelligen Bereich, also grün und insbesondere blau, emittieren.

Gemäß dem Stand der Technik werden in phosphoreszierenden OLEDs als Triplettemitter meist Iridium- und Platinkomplexe eingesetzt. Diese haben jedoch den Nachteil, dass es sich dabei um Metalle mit einer geringen Häufigkeit und daher auch entsprechend um teure Metalle handelt. Zur Schonung der natürlichen Ressourcen dieser Metalle wäre es daher wünschenswert, Emitter auf Basis anderer Metalle zur Verfügung zu haben. Ein weiterer Nachteil der üblicherweise verwendeten Iridium- und Platinkomplexe besteht darin, dass es sich meist um organometallische Komplexe mit Metall-Kohlenstoff-Bindungen handelt. Solche Metall-Kohlenstoff-Bindungen sind teilweise nur schwierig synthetisch zugänglich. Weiterhin weisen diese Komplexe teilweise nur eine geringe thermische Stabilität auf.

Auch bei Matrixmaterialien, beispielsweise Materialien auf Basis von Zinkkomplexen, oder Elektronenleitern, beispielsweise auf Basis von Aluminiumkomplexen, sind noch weitere Verbesserungen wünschenswert.

Aufgabe der vorliegenden Erfindung ist daher die Bereitstellung von Metallkomplexen, welche sich als Emitter, als Matrixmaterialien, als Elektronenleiter oder in anderen Funktionen für die Verwendung in OLEDs eignen, welche eine hohe thermische Stabilität aufweisen, welche bei Verwendung in OLEDs zu hohen Effizienzen und/oder hohen Lebensdauern führen und/oder welche synthetisch einfach zugänglich sind.

WO 2011/066898 offenbart Kupferkomplexe, die einen tripodalen Liganden enthalten, welcher über drei Stickstoffatome von Pyridingruppen an das Kupfer koordiniert. Die Komplexe weisen eine Cyanogruppe als zusätzlichen monodentaten Liganden auf.

WO 2004/081017 offenbart sechsfach koordinierte Al- und Au-Komplexe mit tripodalen Liganden, wobei der Ligand über drei Stickstoffatome von Pyridingruppen und drei Sauerstoff- bzw. Schwefelgruppen von (Thio)Phenolat-gruppen an das Metall koordiniert.

Fry et al. (Inorganica Chimica Acta 2008, 361, 1100) offenbaren geladene Kupferkomplexe mit tripodalen Liganden, in dem drei Pyridingruppen über eine Alkylengruppe an ein verknüpfendes Stickstoffatom binden, welches ebenfalls an das Kupfer koordiniert ist. Mit diesen Komplexen werden Elektronentransferreaktionen untersucht.

Malachowski et al. (J. Chem. Soc. Dalton Trans. 1995, 31) offenbaren geladene Kupferkomplexe mit tridentatem Liganden, in denen drei Pyridin- oder Pyrazolgruppen über Alkylengruppen an ein verknüpfendes Stickstoffatom binden, wobei die Komplexe noch einen weiteren monodentaten Liganden aufweisen. Diese Komplexe werden als Enzymmodelle für Oxidationsreaktionen verwendet.

Lim et al. (Inorg. Chem. 1998, 57, 4898) offenbaren Kupferkomplexe mit tripodalem Liganden, in welchen noch ein weiterer Ligand an das Kupfer koordiniert ist, so dass die Komplexe fünffach koordiniert sind. Diese Komplexe werden als Enzymmodelle untersucht.

Maiti et al. (J. Am. Chem. Soc. 2007, 129, 6998) offenbaren einen geladenen Kupferkomplex mit tripodalem Liganden, in dem drei Pyridingruppen über Methylengruppen an ein verknüpfendes Stickstoffatom binden, welches ebenfalls an das Kupfer koordiniert ist. Mit diesem Komplex als Enzymmodell werden Oxidationsreaktionen untersucht.

Karlin et al. (Inorganica Chimica Acta 1985, 107, L17) offenbaren Kupferkomplexe mit tripodalem Liganden, welche noch ein oder zwei zusätzliche monodentate Liganden aufweisen.

Überraschend wurde gefunden, dass bestimmte, unten näher beschriebene Kupferchelatkomplexe diese Aufgabe lösen und sich sehr gut für die Verwendung in organischen Elektrolumineszenzvorrichtungen eignen, insbesondere bei der Verwendung als emittierendes Material. Dabei zeigen sie eine hohe Lebensdauer, eine hohe Effizienz und/oder eine gute Stabilität gegenüber Temperaturbelastung. Weiterhin ist das Zentralatom dieser Komplexe nicht Iridium oder Platin als seltene Metalle. Ein weiterer Vorteil dieser Komplexe ist, dass diese synthetisch einfach zugänglich sind. Diese Komplexe sowie elektronische Vorrichtungen, insbesondere organische Elektrolumineszenzvorrichtungen, welche diese Komplexe enthalten, sind daher der Gegenstand der vorliegenden Erfindung.

Gegenstand der vorliegenden Erfindung ist somit eine elektrisch neutrale Verbindung gemäß der folgenden Formel (1), 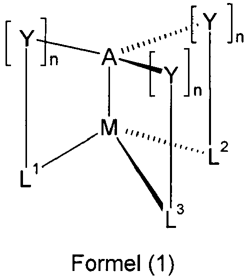 wobei für die verwendeten Symbole und Indizes gilt:
- M: ist Cu(I);
- A: ist ausgewählt aus N oder P;
- Y: ist bei jedem Auftreten gleich oder verschieden eine bivalente Gruppe, ausgewählt aus CR₂, 1,2-Phenylen oder eine orthoverknüpfte Heteroarylengruppe mit 5 oder 6 aromatischen Ringatomen, wobei diese Gruppen jeweils mit einem oder mehreren Resten R substituiert sein kann;
- L¹, L², L³: ist gleich oder verschieden bei jedem Auftreten eine Heteroarylgruppe mit 5 bis 14 aromatischen Ringatomen, die mit einem oder mehreren Resten R substituiert sein kann und die ein Stickstoff, Schwefel- oder Sauerstoffatom enthält, welches an M koordinieren kann, oder eine Aryl- oder Heteroarylgruppe mit 5 bis 14 aromatischen Ringsatomen, die mit einem oder mehreren Resten R substituiert sein kann und die ein exocyclisches Donoratom, ausgewählt aus N, O, S oder P, aufweist, welches an M koordiniert und welches durch einen oder mehrere Reste R substituiert sein kann;
- n: ist bei jedem Auftreten gleich oder verschieden 0, 1 oder 2;
- R: ist bei jedem Auftreten gleich oder verschieden H, D, F, Cl, Br, I, N(R¹)₂, CN, NO₂ OR¹, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenyl- oder Alkinylgruppe mit 2 bis 40 C-Atomen, die jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=NR¹, P(=O)(R¹), SO, SO₂, NR¹, O, S oder CONR¹ ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy-, Heteroaryloxy-, Aralkyl- oder Heteroaralkylgruppe mit 5 bis 60 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei oder mehrere Substituenten R auch miteinander ein mono- oder polycyclisches, aliphatisches, aromatisches, heteroaromatisches und/oder benzoannelliertes Ringsystem bilden;
- R¹: ist bei jedem Auftreten gleich oder verschieden H, D, F, Cl, Br, I, N(R²)₂, CN, NO₂ OH, Si(R²)₃, B(OR²)₂, C(=O)R², P(=O)(R²)₂, S(=O)R², S(=O)2R², OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenyl- oder Alkinylgruppe mit 2 bis 40 C-Atomen, die jeweils mit einem oder mehreren Resten R² substituiert sein kann, wobei eine oder mehrere nicht benachbarte CH₂-Gruppen durch R²C=CR², C=C, Si(R²)₂, C=O, C=S, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy-, Heteroaryloxy-, Aralkyl- oder Heteroaralkylgruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann; dabei können zwei oder mehrere Substituenten R¹ auch miteinander ein mono- oder polycyclisches, aliphatisches, aromatisches, heteroaromatisches und/oder benzoannelliertes Ringsystem bilden;
- R²: ist bei jedem Auftreten gleich oder verschieden H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R² auch miteinander ein mono- oder polycyclisches aliphatisches, aromatisches, heteroaromatisches und/oder benzoannelliertes Ringsystem bilden.

Der Ligand L koordiniert über das freie Elektronenpaar des Brückenkopfes A sowie über die Teilliganden L¹, L² und L³ an das Metall M. Unter einem Teilliganden im Sinne der vorliegenden Erfindung werden in dem Liganden der Formel (2) die Gruppen L¹, L² und L³ verstanden, welche jeweils an das Metall M koordinieren und über A und gegebenenfalls über Y miteinander verknüpft sind. Dabei können die Teilliganden L¹, L² bzw. L³ über ein neutrales oder ein negativ geladenes Donoratom an M koordinieren, wobei dieses Donoratom entweder Teil der Heteroarylgruppe sein kann oder exocyclisch an eine Aryl- oder Heteroarylgruppe gebunden sein kann.

Unter einem Donoratom im Sinne der vorliegenden Erfindung wird ein Atom verstanden, welches mindestens ein freies Elektronenpaar aufweist und dadurch in der Lage ist, an ein Metallion zu binden. Dabei kann das Donoratom neutral oder negativ geladen sein. Beispiele für Donoratome, die Teil einer Heteroarylgruppe sind, sind Stickstoff im Pyrrol, Indol oder Pyridin, Sauerstoff im Furan oder Benzofuran und Schwefel im Thiophen oder Benzothiophen.

Unter einem exocyclischen Donoratom im Sinne dieser Erfindung wird ein Donoratom verstanden, welches nicht Teil einer Aryl- oder Heteroarylgruppe ist, sondern welches als Substituent an eine Aryl- oder Heteroarylgruppe gebunden ist und welches mindestens ein freies Elektronenpaar aufweist und dadurch in der Lage ist, an ein Metallion zu binden. Dabei kann das Donoratom neutral oder negativ geladen sein. Beispiele für exocyclische Donoratome sind Sauerstoff in Form eines Phenols bzw. Phenolats, Schwefel in Form eines Thiols bzw. Thiolats, Stickstoff in Form eines Amins, Imins, Amids oder Imids und Phosphor in Form eines Phosphins.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden. Ein cyclisches Carben im Sinne dieser Erfindung ist eine cyclische Gruppe, welche über ein neutrales C-Atom an das Metall bindet. Dabei kann die cyclische Gruppe gesättigt oder ungesättigt sein. Bevorzugt sind hierbei Arduengo-Carbene, also solche Carbene, bei welchen an das Carben-C-Atom zwei Stickstoffatome gebunden sind.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl und 2,2,2-Trifluorethyl verstanden. Unter einer Alkenylgruppe werden bevorzugt die Reste Ethenyl, Propenyl, Butenyl, Pentenyl, Cyclopentenyl, Hexenyl, Cyclohexenyl, Heptenyl, Cycloheptenyl, Octenyl und Cyclooctenyl verstanden. Unter einer Alkinylgruppe werden bevorzugt Ethinyl, Propinyl, Butinyl, Pentinyl, Hexinyl, Heptinyl und Octinyl verstanden. Unter einer C₁- bis C₄₀-Alkoxygruppe werden bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Benzanthracen, Benzphenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die Teilliganden L¹, L² und L³ können auch über Reste R, die miteinander verbrückt sind, miteinander verbunden sein. Dadurch ergeben sich aus dem Komplex der Formel (1), der einen polypodalen Liganden aufweist, Kryptate. Unter einem polypodalen Liganden im Sinne dieser Erfindung wird ein Ligand verstanden, in welchem drei koordinierende Teilliganden L¹, L² und L³ durch eine Gruppe A aneinander gebunden sind. Bei dem Liganden der Verbindung der Formel (1) handelt es sich daher um einen polypodalen Liganden. Unter einem Kryptat im Sinne dieser Erfindung wird eine Verbindung zwischen einem Kryptanden und einem Metallion verstanden, in der das Metallion von den Brücken des komplexbildenden Kryptanden dreidimensional umgeben ist. Unter einem Kryptanden im Sinne dieser Erfindung wird ein makrocyclischer tripodaler Ligand verstanden. Dabei kann ein Kryptand entstehen, wenn alle drei der Teilliganden L¹, L² und L³ oder wenn zwei der drei Teilliganden über Reste R miteinander verknüpft sind. Dies ist im Folgenden schematisch dargestellt: 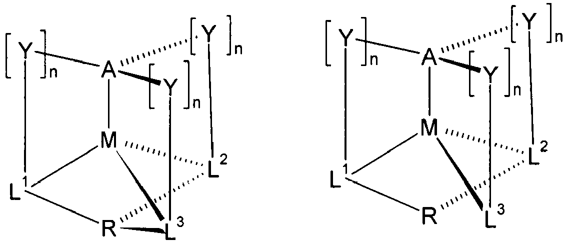 wobei R in dieser Struktur die Ringbildung von Resten R an den Teilliganden L¹, L² und L³ darstellt und die weiteren verwendeten Symbole und Indizes die oben genannten Bedeutungen aufweisen. Dabei weist R die oben genannten Bedeutungen auf, wobei es sich um eine bivalente bzw. trivalente Gruppe handelt, so dass die entsprechenden monovalenten Gruppen wie Halogene nicht in Frage kommen.

Die Verbindung der Formel (1) ist elektrisch neutral. Dies wird dadurch erreicht, dass die Ladungen der Teilliganden L¹, L² und L³ so gewählt werden, dass sie die Ladung von M kompensieren. Dabei koordiniert bevorzugt einer der Teilliganden L¹, L² oder L³ über ein negativ geladenes Donoratom und die anderen Teilliganden koordinieren über neutrale Donoratome.

In einer bevorzugten Ausführungsform der Erfindung enthält der Cyclus, der aus A, Y, M und L¹ bzw. L² bzw. L³ gebildet wird, 5, 6, 7, 8 oder 9 Ringatome. Abhängig von der genauen Struktur der Teilliganden L¹, L² bzw. L³ wird der Index n entsprechend so gewählt, dass durch die Koordination von M ein 5-, 6-, 7-, 8- oder 9-gliedriger Ring entsteht. Was unter der Bildung eines 5-, 6-, 7-, 8- oder 9-gliedrigen Rings verstanden wird, ist im Folgenden an einigen Teilstrukturen schematisch dargestellt:

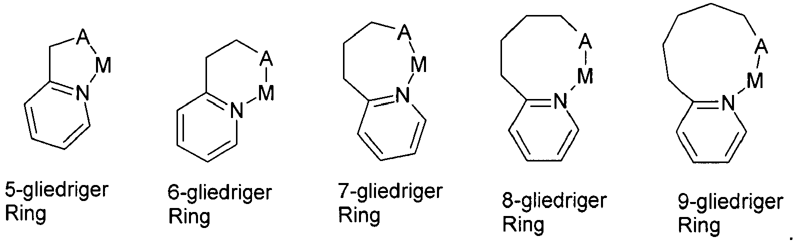

Dabei ist jeweils die Struktur enthaltend einen Arm des polypodalen Liganden und einen Teilliganden abgebildet. In allen abgebildeten Strukturen ist der Teilligand Pyridin und Y steht für CH₂. Für den 5-gliedrigen Ring ist n = 1, für den 6-gliedrigen Ring ist n = 2, etc.. Es lassen sich bei anderer Wahl der Teilliganden auch beispielsweise mit n = 0 ein 5-gliedriger Ring oder mit n = 0 oder 1 ein sechsgliedriger Ring bilden, wie im Folgenden schematisch dargestellt:

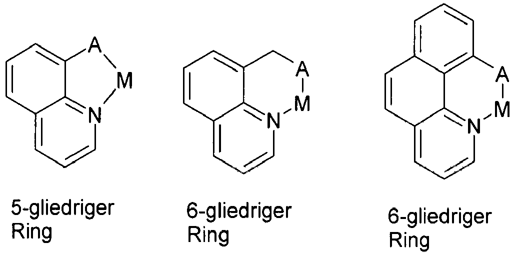

Dabei ist in der ersten Struktur der Teilligand Chinolin und n = 0, in der zweiten Struktur der Teilligand Chinolin, n = 1 und Y = CH₂ und in der dritten Struktur der Teilligand Phenanthridin und n = 0.

Besonders bevorzugt enthält der Cyclus, der aus A, Y, M und L¹ bzw. L² bzw. L³ gebildet wird, 6, 7, 8 oder 9 Ringatome, besonders bevorzugt 6, 7 oder 8 Ringatome. Welche Kombinationen an Ringgrößen bevorzugt sind für die einzelnen Teilliganden L¹ bzw. L² bzw. L³, hängt von der Natur von Y ab. Wenn Y eine aromatische Gruppe ist oder wenn n = 0 ist und der Teilligand L¹ bzw. L² bzw. L³ direkt an A bindet, sind bevorzugte Ringgrößen (6-6-6), (5-6-7), (6-6-7), (6-7-7) und (7-7-7). Dabei gibt jede dieser drei Zahlen die Ringgröße an, die der entsprechende "Arm" des polypodalen Liganden mit dem Metallatom bildet. So bedeutet beispielsweise (5-6-7), dass der Cyclus, der aus A, Y, M und L¹ gebildet wird, 5 Ringatome und der Cyclus, der aus A, Y, M und L² gebildet wird, 6 Ringatome und der Cyclus, der aus A, Y, M und L³ gebildet wird, 7 Ringatome aufweist. Wenn Y aliphatisch ist, sind die einzelnen Arme des polypodalen Liganden nicht planar und dadurch etwas verkürzt im Vergleich zu rein aromatischen Armen, so dass die Ringgrößen (6-6-6) hier nur zu einem verzerrten Tetraeder führen und größere Ringe von Vorteil sind, insbesondere die Kombination aus 6-gliedrigen mit 7- und/oder 8-gliedrigen Ringen oder Kombinationen aus 7- und/oder 8-gliedrigen Ringen.

In einer bevorzugten Ausführungform der Erfindung weist L¹ bzw. L² bzw. L³ 5 bis 13 aromatische Ringatome auf, ganz besonders bevorzugt 5 bis 10 aromatische Ringatome. Dabei können die Aryl- bzw. Heteroarylgruppen durch einen oder mehrere Reste R substituiert sein, wie oben beschrieben.

In einer bevorzugten Ausführungsform der Erfindung sind L¹, L² und L³ gleich oder verschieden bei jedem Auftreten ausgewählt aus den Gruppen der Formeln (2) bis (7) und (9) bis (41): 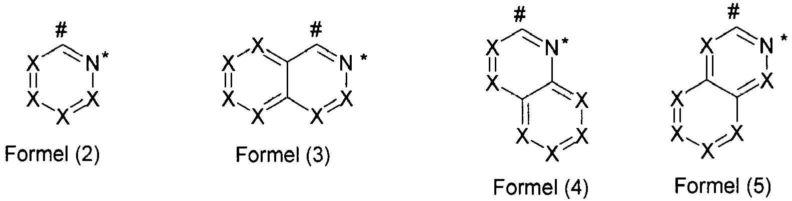 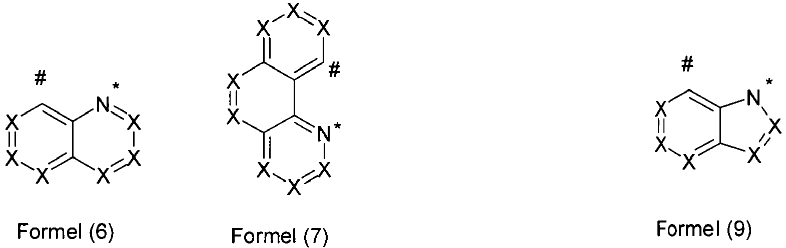 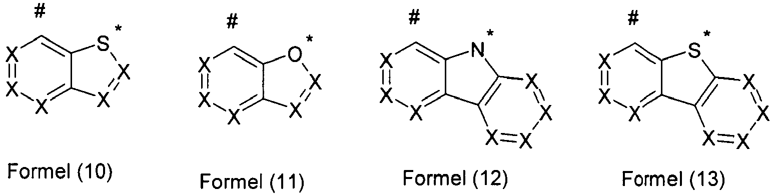 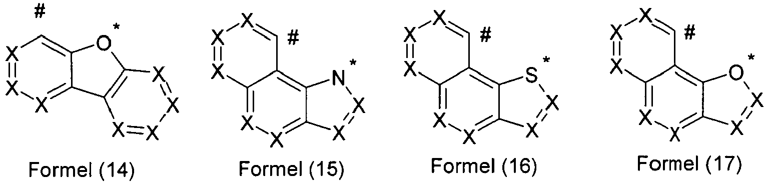 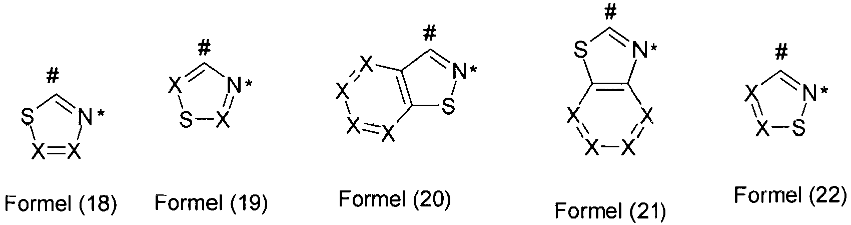 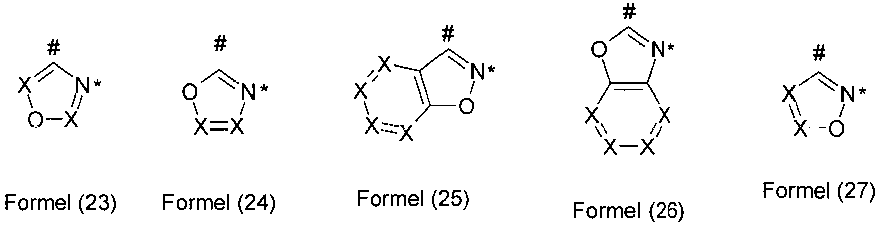 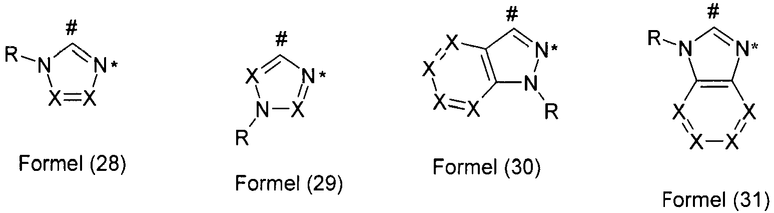 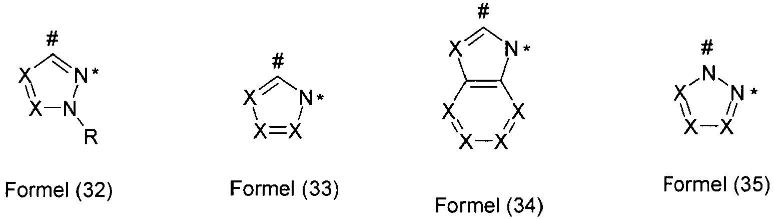 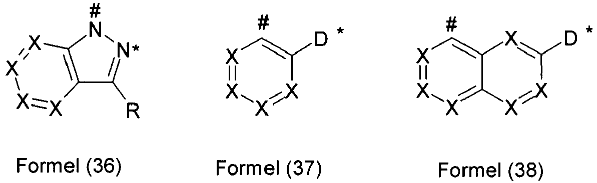 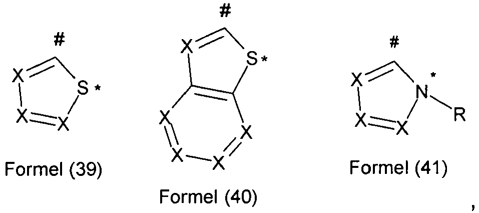 wobei R dieselbe Bedeutung hat, wie oben beschrieben, und weiterhin gilt:
- X: steht bei jedem Auftreten gleich oder verschieden für CR oder N;
- D: steht bei jedem Auftreten gleich oder verschieden für OH, O- SH, S⁻, NR₂, NR-, PR-, PR₂, OR, SR, COO-, -C(=O)R, -CR(=NR) oder -N(=CR₂).

Bevorzugt sind unter diesen Strukturen die Gruppen der Formeln (2), (7), (9), (10), (12), (13), (15), (33), (34), (37), (39), (40) und (41) und besonders bevorzugt sind die Gruppen der Formeln (9), (12), (33), (34), (39), (40) und (41).

Dabei koordinieren die Gruppen der Formeln (2) bis (7) und (9) bis (41) an das Metall M über die durch * gekennzeichnete Position. Die durch # gekennzeichnete Position gibt die Position an, an der der Teilligand L¹ bzw. L² bzw. L³ an Y bzw. an A gebunden ist.

Bevorzugt stehen maximal drei Symbole X in jeder Gruppe für N, besonders bevorzugt stehen maximal zwei Symbole X in jeder Gruppe für N, ganz besonders bevorzugt steht maximal ein Symbol X in jeder Gruppe für N. Insbesondere bevorzugt stehen alle Symbole X für CR.

In einer weiteren bevorzugten Ausführungsform der Erfindung ist Y gleich oder verschieden bei jedem Auftreten CR₂.

Bevorzugte Strukturen gemäß Formel (1) sind Strukturen, in denen die oben genannten Bevorzugungen gleichzeitig auftreten, also Strukturen, in denen gilt:
- L¹, L², L³: ist gleich oder verschieden bei jedem Auftreten ausgewählt aus den oben genannten Gruppen gemäß den Formeln (2) bis (7) oder (9) bis (41), wobei maximal drei Symbole X in jeder Gruppe für N stehen;
- Y: ist gleich oder verschieden bei jedem Auftreten CR₂;
- n: ist gleich oder verschieden bei jedem Auftreten 0, 1 oder 2.

Besonders bevorzugte Strukturen gemäß Formel (1) sind Strukturen, in denen gilt:
- L¹, L², L³: ist gleich oder verschieden bei jedem Auftreten ausgewählt aus den oben genannten Gruppen gemäß den Formeln (2), (7), (9), (10), (12), (13), (15), (33), (34), (37), (39), (40) oder (41), wobei maximal zwei Symbole X, bevorzugt maximal ein Symbol X in jeder Gruppe für N stehen;
- Y: ist gleich oder verschieden bei jedem Auftreten CR₂;
- n: ist gleich oder verschieden bei jedem Auftreten 0 oder 1.

Die weiteren verwendeten Symbole und Indizes haben jeweils die oben genannten Bedeutungen.

Bevorzugt sind weiterhin Verbindungen gemäß Formel (1) bzw. gemäß den oben aufgeführten bevorzugten Ausführungsformen, in denen R bei jedem Auftreten gleich oder verschieden für H, D, F, CN, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, die jeweils mit einem oder mehreren Resten R¹ substituiert sein kann, wobei eine oder mehrere nicht benachbarte CH₂-Gruppen durch R¹C=CR¹, 0 oder S ersetzt sein können und ein oder mehrere H-Atome durch D oder F ersetzt sein können, oder ein aromatisches oder heteroaromatisches Ringsystem mit 5 bis 18 aromatischen Ringatomen, welche jeweils durch einen oder mehrere Reste R¹ substituiert sein kann, oder eine Diarylaminogruppe mit 10 bis 20 aromatischen Ringatomen, welche durch einen oder mehrere Reste R¹ substituiert sein kann, oder eine Kombination dieser Systeme steht; dabei können zwei oder mehrere Substituenten R auch miteinander ein mono- oder polycyclisches aliphatisches, aromatisches und/oder benzoannelliertes Ringsystem bilden. Besonders bevorzugt steht das Symbol R in diesen Verbindungen, gleich oder verschieden bei jedem Auftreten, für H, D, F, eine geradkettige Alkylgruppe mit 1 bis 6 C-Atomen oder eine verzweigte Alkylgruppe mit 3 bis 6 C-Atomen, die jeweils mit einem oder mehreren Resten R¹ substituiert sein kann, wobei ein oder mehrere H-Atome durch F ersetzt sein können, oder eine Arylgruppe mit 6 bis 10 aromatischen Ringatomen oder ein aromatisches Ringsystem mit 12 bis 18 aromatischen Ringatomen, welche jeweils durch einen oder mehrere Reste R¹ substituiert sein kann; dabei können zwei oder mehrere Substituenten R auch miteinander ein mono- oder polycyclisches aliphatisches, aromatisches und/oder benzoannelliertes Ringsystem bilden.

Weiterhin bevorzugt sind Verbindungen, in denen mindestens zwei der Teilliganden L¹, L² und L³ gleich sind und auch gleich substituiert sind.

Beispiele für geeignete Liganden, die als monoanionische Liganden an M koordinieren, sind die im Folgenden aufgeführten Liganden, wobei sie bei Koordination als monoanionische Liganden jeweils in deprotonierter Form vorliegen.

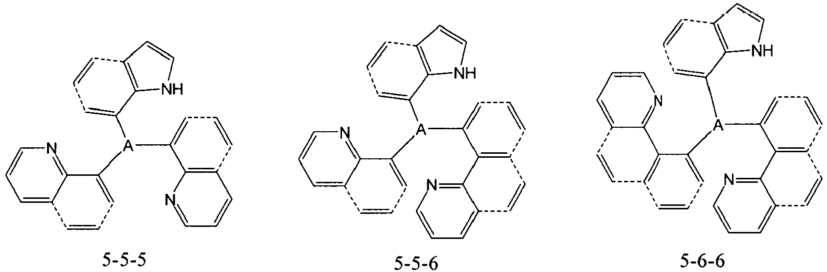

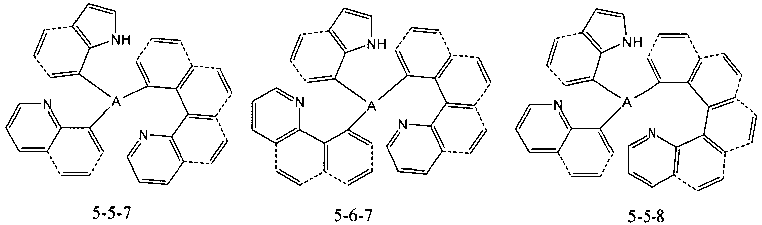

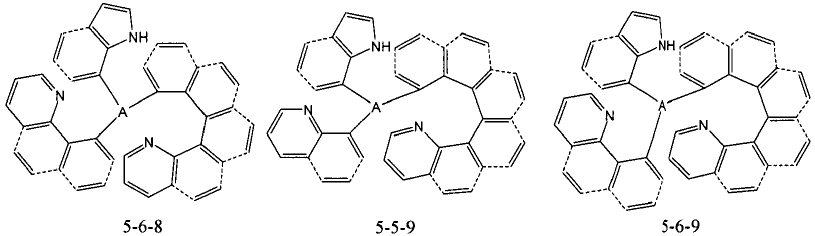

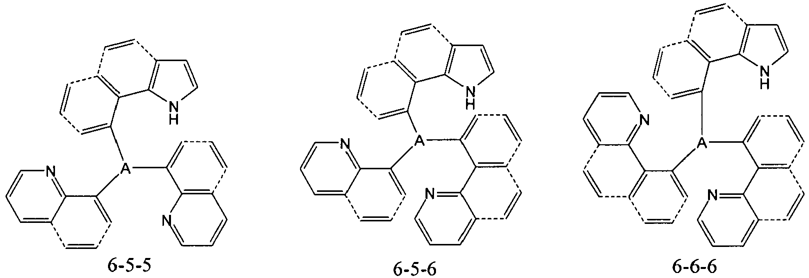

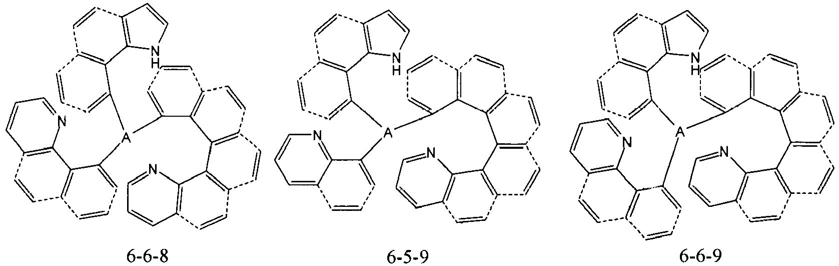

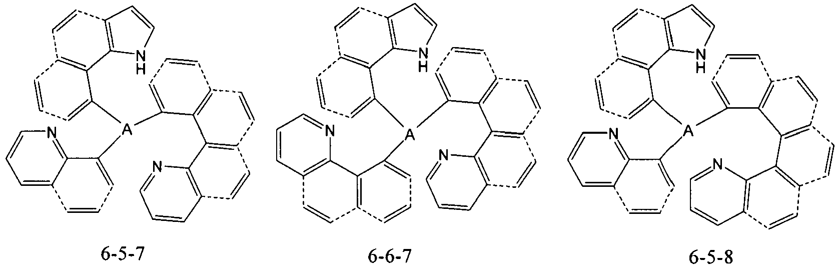

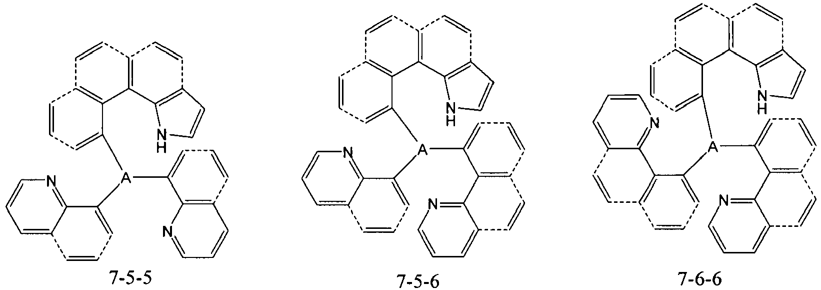

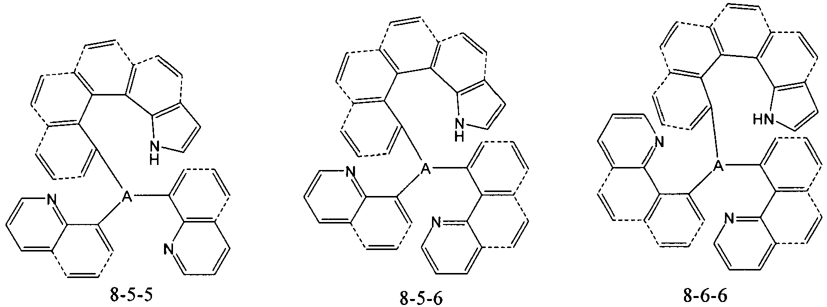

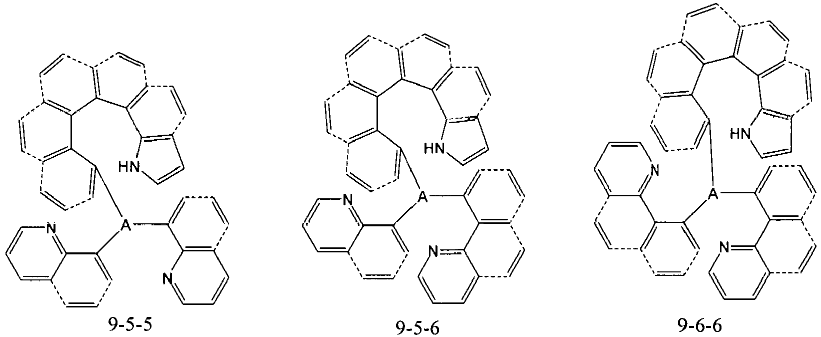

Weitere Beispiele für geeignete Liganden, die als monoanionische Liganden an M koordinieren, sind die im Folgenden aufgeführten Liganden, wobei sie bei Koordination als monoanionische Liganden jeweils in einfach deprotonierter Form vorliegen.

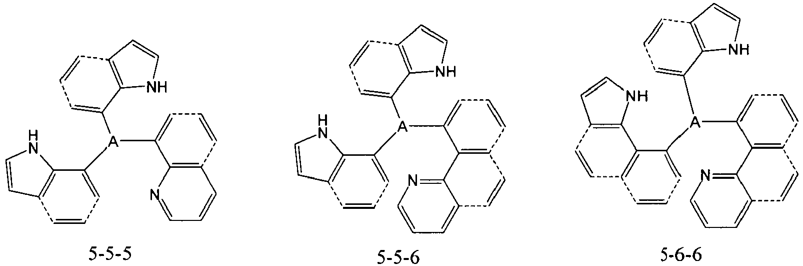

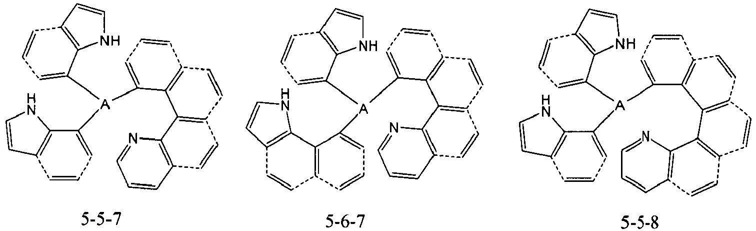

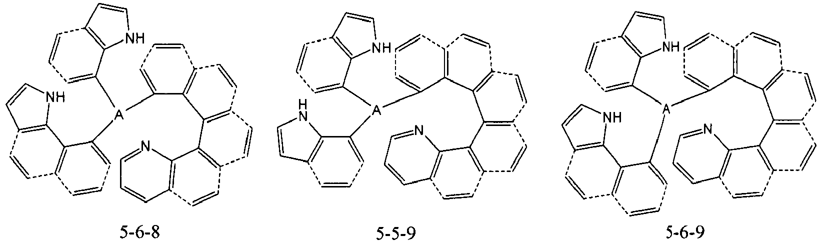

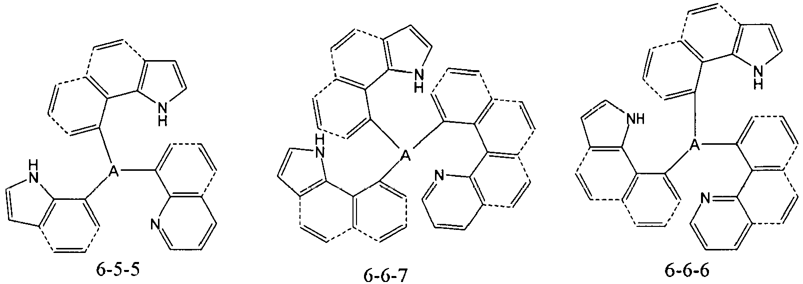

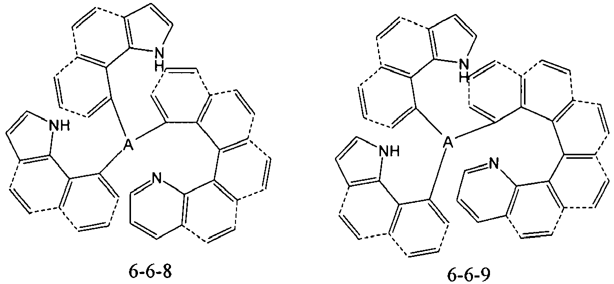

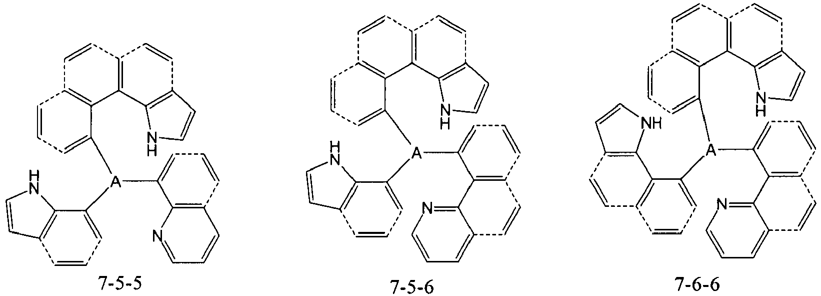

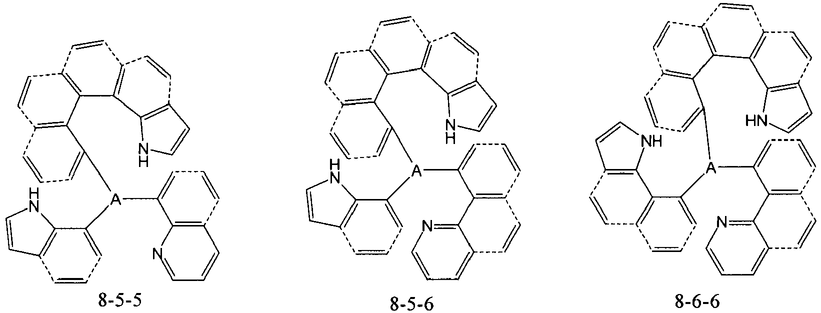

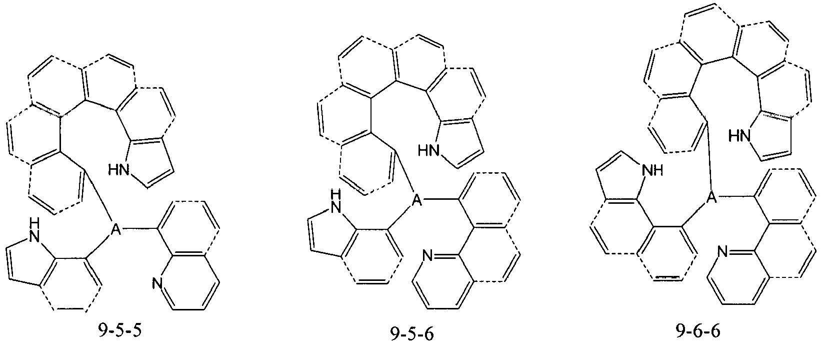

Weitere Beispiele für geeignete Liganden, die als monoanionische Liganden an M koordinieren, sind die im Folgenden aufgeführten Liganden, wobei sie bei Koordination als monoanionische Liganden jeweils in einfach deprotonierter Form vorliegen.

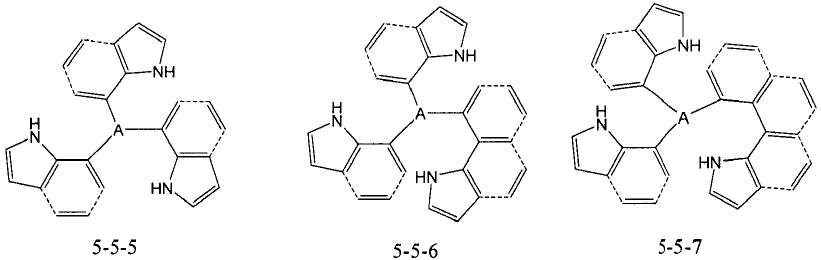

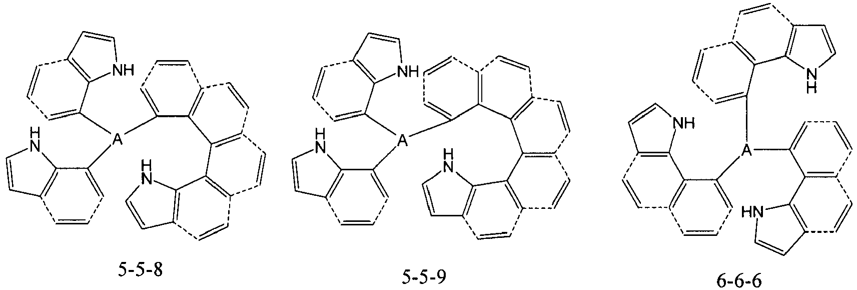

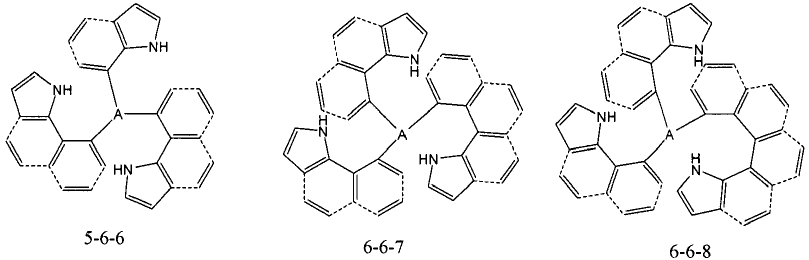

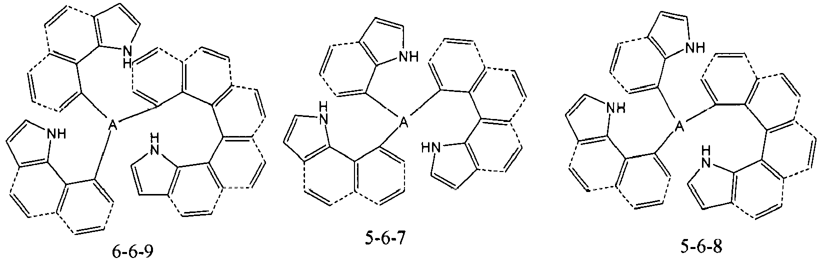

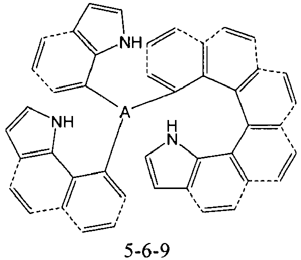

Wie oben beschrieben, wird für die Metallkomplexe ein Ligand gewählt, der die Ladung des Metallatoms ausgleicht, also ein monoanionischer Ligand. A steht in den oben aufgeführten Strukturen für N oder P. Die durch gestrichelte Bindungen angedeuteten Aromaten können unabhängig voneinander vorhanden sein oder auch nicht, jedoch nur, wenn sie eine vollständige aromatische Gruppe bilden. Weiterhin können einzelne C-Atomen in den oben aufgeführten Liganden auch durch N ersetzt sein. Weiterhin können die Strukturen durch einen oder mehrere Reste R substituiert sein, wobei R die oben genannten Bedeutungen aufweist. In den oben abgebildeten Liganden werden diese der Anschaulichkeit halber mit einer dreistelligen Zahl benannt. Jede dieser drei Zahlen gibt die Ringgröße an, die der entsprechende "Arm" des polypodalen Liganden mit dem Metallatom bildet.

Beispiele für erfindungsgemäße Verbindungen gemäß Formel (1) sind die im Folgenden abgebildeten Verbindungen.

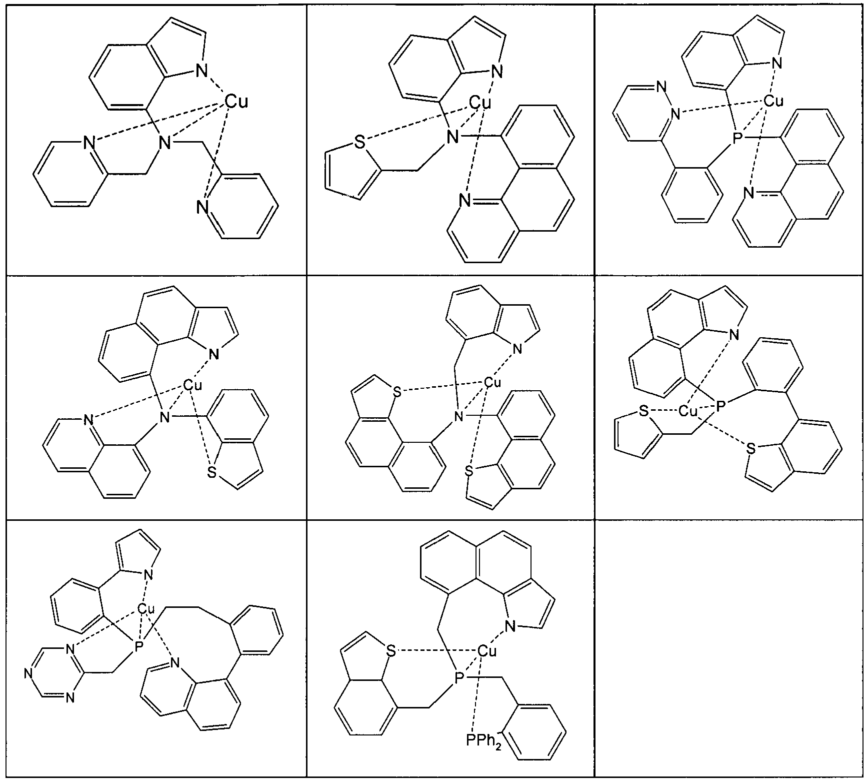

Die Synthese der Liganden kann prinzipiell für alle Liganden analog durchgeführt werden, wobei jeweils ein tertiäres Amin bzw. Phosphin synthetisiert wird. Dies kann auf verschiedene Arten erfolgen.

Eine Möglichkeit ist die dreifache Aminierung von Ammoniak, wie im folgenden Schema 1 allgemein für die Synthese eines tertiären Amins mit zwei unterschiedlichen Armen dargestellt.

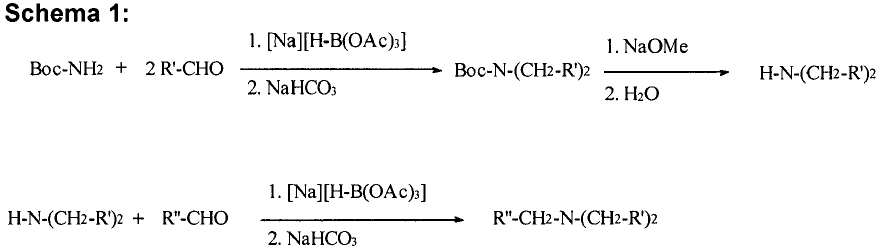

Hierzu wird mono-geschützter Ammoniak mit zwei Äquivalenten eines Aldehyds unter reduzierenden Bedingungen umgesetzt, wobei statt Boc auch andere Schutzgruppen eingesetzt werden können. Dabei kann die Aminierung analog zu A. Beni et al. (Chem. Eur. J. 2008, 14, 1804-1813) durchgeführt werden. Das Reaktionsprodukt wird entschützt und mit einem weiteren Aldehyd, der gleich oder verschieden dem ersten Aldehyd sein kann, unter reduzierenden Bedingungen umgesetzt. So ist die Synthese eines Liganden mit zwei gleichen Armen und einem davon verschiedenen Arm oder mit drei gleichen Armen möglich. Entsprechend ist unter Verwendung von zwei unterschiedlichen Amin-Schutzgruppen mit unterschiedlichen Abspaltungsbedingungen auch die Synthese von Liganden mit drei unterschiedlichen Armen möglich. Durch Hartwig-Buchwald Kreuzkupplungsreaktionen können entsprechend aromatische Analoga synthetisiert werden.

Eine weitere Möglichkeit für die Darstellung eines sekundären Amins ist beispielsweise die Umsetzung eines Ethen-Derivats mit Ammoniumchlorid, wie im folgenden Schema 2 allgemein dargestellt (Umsetzung analog zu K. Ladomenou et al., Tetrahedron 2007, 63, 2882-2887). Dieses sekundäre Amin kann dann beispielsweise analog zur in Schema 1 gezeigten Umsetzung weiter zum tertiären Amin umgesetzt werden.

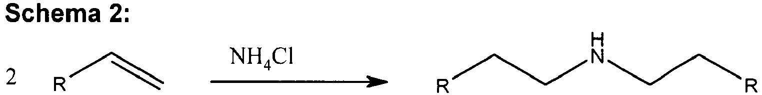

Eine mögliche Synthese von tertiären Phosphinen mit zwei unterschiedlichen Armen ist im folgenden Schema 3 dargestellt.

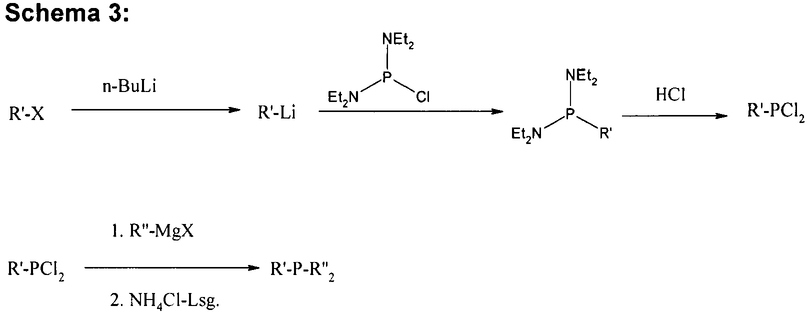

Dabei steht R' bzw. R" gleich oder verschieden bei jedem Auftreten für eine Alkyl-, Aryl- bzw. Heteroarylgruppe gemäß der obigen Definition für R, und X steht für Cl, Br oder I.

Die Synthese kann analog zu A. Tsurusaki et al., Bull. Chem. Soc. Jpn. 2010, 83(5), 456-478 durchgeführt werden. Hierzu wird ein Aryl-, Heteroaryl- oder Alkylhalogenid lithiiert und mit einem zweifach geschützten Phosphinchlorid umgesetzt, wobei statt NEt₂ auch andere Schutzgruppen eingesetzt werden können. Das Reaktionsprodukt wird entschützt zum entsprechenden Phosphindichlorid und mit einem Grignard-Reagenz zum tertiären Phosphin umgesetzt. So ist die Synthese eines Liganden mit zwei gleichen Armen und einem davon verschiedenen Arm oder mit drei gleichen Armen möglich. Entsprechend ist unter Verwendung von zwei unterschiedlichen Phosphin-Schutzgruppen mit unterschiedlichen Abspaltungsbedingungen auch die Synthese von Liganden mit drei unterschiedlichen Armen möglich.

Die Komplexe gemäß Formel (1) bzw. gemäß den oben aufgeführten bevorzugten Ausführungsformen sind prinzipiell durch verschiedene Verfahren herstellbar. Um einen neutralen Metallkomplex zu erhalten, muss die Höhe der Ladung des Liganden die Ladung des Metalls ausgleichen. Der Metallkomplex wird dazu mit dem freien Liganden, gegebenenfalls in deprotonierter Form, umgesetzt.

Dabei kann die Deprotonierung des Liganden in situ durch einen Metallprecursor mit protonierbarem, bevorzugt nicht nukleophilem Gegenion, wie z. B. Mesityl oder Amid, erfolgen. Damit erhält man direkt den neutralen Metallkomplex der Formel (1).

Alternativ kann zunächst der Ligand mit einer Base deprotoniert und dann mit einem Metallsalz umgesetzt werden, welches das Kation des deprotonierten Liganden ersetzt. Man erhält dann direkt den neutralen Metallkomplex.

Eine dritte Möglichkeit besteht in der Umsetzung eines Metallsalzes mit dem nicht deprotonierten Liganden. Hierbei entsteht als Zwischenstufe ein positiv geladener Metallkomplex, der anschließend zum neutralen Komplex deprotoniert werden kann.

Die Synthese von Kryptaten kann beispielsweise durch Umsetzung eines Komplexes mit entsprechendem tripodalen Liganden mit einer verbrückenden Einheit erfolgen, wie an einem Beispiel im folgenden Schema 4 dargestellt.

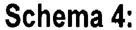

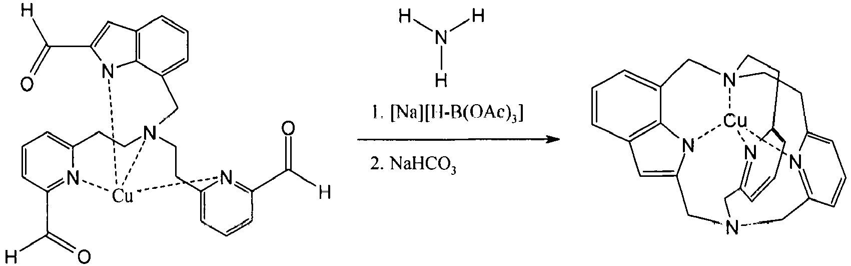

Ein weiterer Gegenstand der vorliegenden Erfindung ist somit ein Verfahren zur Herstellung der erfindungsgemäßen Verbindungen, gekennzeichnet durch die Umsetzung eines Metallsalzes bzw. Metallkomplexes des Metalls M mit dem entsprechenden freien Liganden, gegebenenfalls in deprotonierter Form. Wenn der Ligand nicht in deprotonierter Form eingesetzt wird, wird anschließend an die Komplexierung noch ein Deprotonierungsschritt durchgeführt werden. Insbesondere werden Metallsalze bzw. Metallkomplexe mit protonierbarem, nicht nukleophilem Gegenion eingesetzt.

Beispiele für geeignete Kupferverbindungen, die als Edukte eingesetzt werden können, sind insbesondere Kupfer(I)salze mit schwach koordinierenden Anionen, wie Cu(OAc), Cu₂(CO₃), [Cu(MeCN)₄][BF₄], CuBF₄, [Cu(MeCN)₄][PF₆], Kupfer(I)mesityl oder Kupfer(I)amide, wie z. B. Kupfer(I)-pyrrolidin.

Die Synthese kann auch thermisch, photochemisch oder durch Mikrowellenstrahlung aktiviert werden und/oder im Autoklaven bzw. allgemein unter Druck durchgeführt werden. Durch diese Verfahren lassen sich die Komplexe in hoher Reinheit, bevorzugt in einer Reinheit von > 99% nach ¹H-NMR oder HPLC, erhalten.

Für die Verarbeitung aus Lösung, beispielsweise durch Spin-Coating oder durch Druckverfahren, sind Lösungen bzw. Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis-(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein oder mehrere Lösemittel sein, insbesondere ein oder mehrere der oben genannten Lösemittel. Wie solche Lösungen hergestellt werden können, ist dem Fachmann bekannt und beispielsweise in der WO 2002/072714, der WO 2003/019694 und der darin zitierten Literatur beschrieben. Die weitere Verbindung kann aber auch eine weitere organische oder anorganische Verbindung sein, die ebenfalls in der elektronischen Vorrichtung eingesetzt wird, beispielsweise ein Matrixmaterial. Diese weitere Verbindung kann auch polymer sein.

Die oben beschriebenen Verbindungen gemäß Formel (1) bzw. die oben aufgeführten bevorzugten Ausführungsformen können in einer elektronischen Vorrichtung als aktive Komponente verwendet werden. Ein weiterer Gegenstand der vorliegenden Erfindung ist daher die Verwendung einer Verbindung nach Formel (1) bzw. nach einer der bevorzugten Ausführungsformen in einer elektronischen Vorrichtung. Weiterhin können die erfindungsgemäßen Verbindungen zur Erzeugung von Singulett-Sauerstoff, in der Photokatalyse oder in Sauerstoffsensoren eingesetzt werden.

Nochmals ein weiterer Gegenstand der vorliegenden Erfindung ist eine elektronische Vorrichtung enthaltend mindestens eine Verbindung gemäß Formel (1) bzw. nach einer der bevorzugten Ausführungsformen.

Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine Verbindung der oben aufgeführten Formel (1) enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser), enthaltend in mindestens einer Schicht mindestens eine Verbindung gemäß der oben aufgeführten Formel (1). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Die erfindungsgemäßen Verbindungen zeigen besonders gute Eigenschaften als Emissionsmaterial in organischen Elektrolumineszenzvorrichtungen. Eine bevorzugte Ausführungsform der Erfindung sind daher organische Elektrolumineszenzvorrichtungen.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Eine bevorzugte Ausführungsform sind Dreischichtsysteme, wobei die drei Schichten blaue, grüne und orange oder rote Emission zeigen (siehe z. B. WO 2005/011013) bzw. Systeme, welche mehr als drei emittierende Schichten aufweisen. Eine weitere bevorzugte Ausführungsform sind Zweischichtsysteme, wobei die beiden Schichten entweder blaue und gelbe oder blaugrüne und orange Emission zeigen. Zweischichtsysteme sind insbesondere für Beleuchtungsanwendungen von Interesse. Solche Ausführungsformen sind mit den erfindungsgemäßen Verbindungen besonders geeignet, da diese häufig gelbe bzw. orange Emission zeigen. Die weiß emittierenden Elektrolumineszenzvorrichtungen können für Beleuchtungsanwendungen oder als Backlight für Displays oder mit Farbfilter als Display eingesetzt werden.

In einer bevorzugten Ausführungsform der Erfindung enthält die organische Elektrolumineszenzvorrichtung die Verbindung gemäß Formel (1) bzw. die oben aufgeführten bevorzugten Ausführungsformen als emittierende Verbindung in einer oder mehreren emittierenden Schichten.

Wenn die Verbindung gemäß Formel (1) als emittierende Verbindung in einer emittierenden Schicht eingesetzt wird, wird sie bevorzugt in Kombination mit einem oder mehreren Matrixmaterialien eingesetzt. Die Mischung aus der Verbindung gemäß Formel (1) und dem Matrixmaterial enthält zwischen 1 und 99 Vol.-%, vorzugsweise zwischen 2 und 90 Vol.-%, besonders bevorzugt zwischen 3 und 40 Vol.-%, insbesondere zwischen 5 und 15 Vol.-% der Verbindung gemäß Formel (1) bezogen auf die Gesamtmischung aus Emitter und Matrixmaterial. Entsprechend enthält die Mischung zwischen 99 und 1 Vol.-%, vorzugsweise zwischen 98 und 10 Vol.-%, besonders bevorzugt zwischen 97 und 60 Vol.-%, insbesondere zwischen 95 und 85 Vol.-% des Matrixmaterials bzw. der Matrixmaterialien bezogen auf die Gesamtmischung aus Emitter und Matrixmaterial.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015, oder verbrückte Carbazolderivate, z. B. gemäß US 2009/0136779, WO 2010/050778, WO 2011/042107 oder WO 2011/088877.

Es kann auch bevorzugt sein, mehrere verschiedene Matrixmaterialien als Mischung einzusetzen. Hierfür eignen sich insbesondere Mischungen aus mindestens einem elektronentransportierenden Matrixmaterial und mindestens einem lochtransportierenden Matrixmaterial oder Mischungen aus mindestens zwei elektronentransportierenden Matrixmaterialien oder Mischungen aus mindestens einem loch- oder elektronentransportierenden Matrixmaterial und mindestens einem weiteren Material mit einer großen Bandlücke, welches somit weitgehend elektrisch inert ist und nicht oder nicht in wesentlichem Umfang am Ladungstransport teilnimmt, wie z. B. in WO 2010/108579 beschrieben.

Weiterhin kann es bevorzugt sein, die erfindungsgemäße Verbindung als Matrixmaterial, insbesondere als Matrixmaterial für Triplettemitter einzusetzen.

Weiterhin kann es bevorzugt sein, die erfindungsgemäße Verbindung als Elektronentransportmaterial oder als Lochblockiermaterial einzusetzen.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lathanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektronen (z. B. Al/Ni/NiOₓ, Al/PtOₓ) bevorzugt sein. Für einige Anwendungen muss mindestens eine der Elektroden transparent sein, um entweder die Bestrahlung des organischen Materials (O-SC) oder die Auskopplung von Licht (OLED/PLED, O-LASER) zu ermöglichen. Ein bevorzugter Aufbau verwendet eine transparente Anode. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

In den weiteren Schichten können generell alle Materialien verwendet werden, wie sie gemäß dem Stand der Technik für die Schichten verwendet werden und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine Verbindung gemäß Formel (1) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf organische Elektrolumineszenzvorrichtungen enthaltend Verbindungen gemäß Formel (1) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch folgende überraschende Vorteile gegenüber dem Stand der Technik aus:
1. Im Gegensatz zu vielen Metallkomplexen gemäß dem Stand der Technik, die der teilweisen oder vollständigen pyrolytischen Zersetzung bei Sublimation unterliegen, weisen die erfindungsgemäßen Verbindungen eine hohe thermische Stabilität auf. Insbesondere die neutralen Verbindungen gemäß der vorliegenden Erfindung weisen dabei eine überraschend niedrige Sublimationstemperatur auf.
2. Organische Elektrolumineszenzvorrichtungen enthaltend die erfindungsgemäßen Verbindungen als emittierende Materialien, als Matrixmaterialien oder als Elektronentransportmaterialien weisen weiten gute Elgenschaften In Bezug auf Effizienz, Betriebsspannung und Lebensdauer auf.
3. Die Verbindungen gemäß Formel (1) basieren nicht auf den seltenen Metallen Iridium oder Platin, was zur Ressourcenschonung dieser Metalle beiträgt.
4. Die Komplexe gemäß Formel (1) bzw. gemäß der oben aufgeführten bevorzugten Ausführungsformen sind gut und in hohen Ausbeuten und hohen Reinheiten synthetisch zugänglich.

Die oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße Komplexe herstellen und diese in organischen elektronischen Vorrichtungen verwenden bzw. das erfindungsgemäße Verfahren anwenden und so die Erfindung im gesamten beanspruchten Bereich ausführen.

### Beschreibung der Figuren:

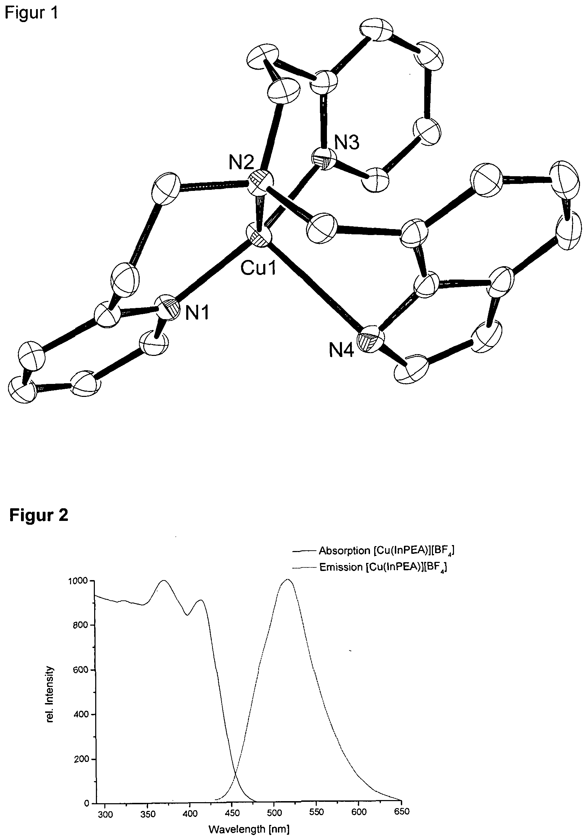

**Figur 1****:** Kristallstruktur von [Cu(InPEA)](BF₄), wobei die verzerrt tetraedrische Koordination des Liganden an das Cu zu erkennen ist (die Protonen und das BF₄-Anion sind der Übersichtlichkeit halber nicht abgebildet).
**Figur 2****:** Absorptions- und Emissionsspektrum von [Cu(InPEA)](BF₄) im Feststoff (Reinstoff; Emissionsmaximum: 519 nm, gelb-grüne Emission).

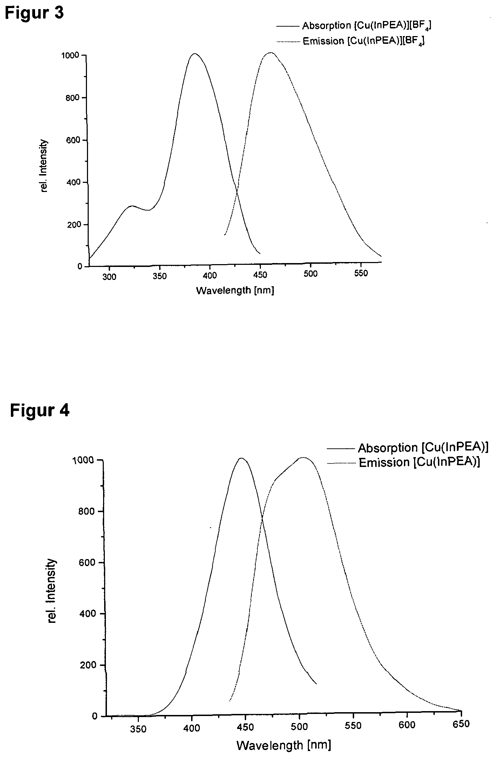

**Figur 3****:** Absorptions- und Emissionsspektrum von [Cu(InPEA)](BF₄) in Lösung in Dichlormethan (Emissionsmaximum: 464 nm, blaue Emission).
**Figur 4****:** Absorptions- und Emissionsspektrum von [Cu(InPEA)] in Lösung in Dichlormethan (Emissionsmaximum: 506 nm, gelbgrüne Emission).

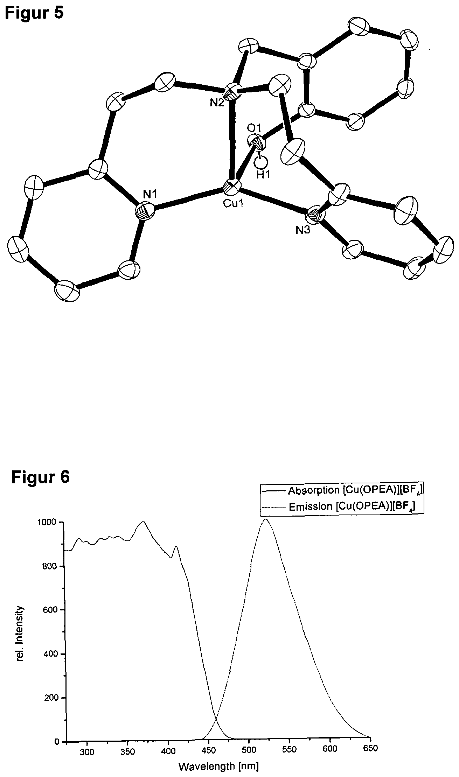

**Figur 5****:** Kristallstruktur von [Cu(OPEA)](BF₄), wobei die verzerrt tetraedrische Koordination des Liganden an das Cu zu erkennen ist (die Protonen und das BF₄-Anion sind der Übersichtlichkeit halber nicht abgebildet).
**Figur 6****:** Absorptions- und Emissionsspektrum von [Cu(OPEA)](BF₄) im Feststoff (Reinstoff; Emissionsmaximum: 525 nm, gelb-grüne Emission).

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden.

### Beispiel 1: Synthese des Liganden InPEA (N-((1H-indol-7-yl)methyl)-2-(pyridin-2-yl)-N-(2-(pyridin-2-yl)ethyl)ethanamin)

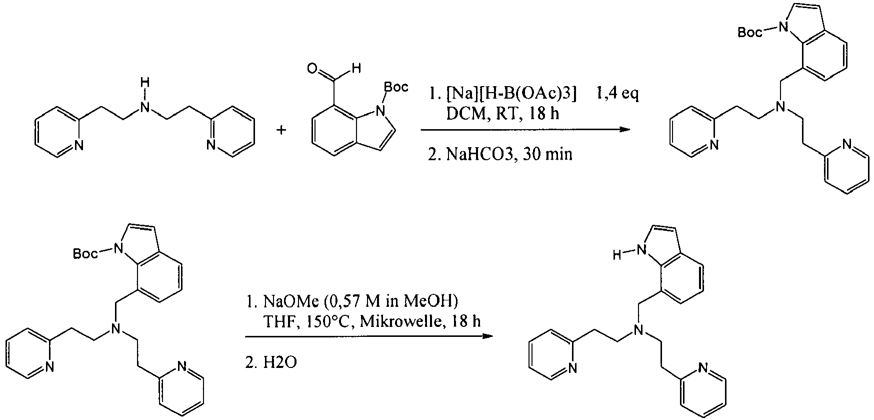

### Schritt 1:

In einem 250 ml Schlenkkolben werden 1,25 g Bis(2-(pyridin-2-yl)ethyl)amin (5,51 mmol) (synthetisiert gemäß K. Ladomenou et al., Tetrahedron 2007, 63, 2882-2887) und 1,35 g N-Boc-indol-7-carbaldehyd (CAS: 597544-14-4) (5,51 mmol) inertisiert und dann in 30 ml Dichlormethan gelöst. Anschließend wird eine inerte Suspension aus 1,63 g Na-triacetoxyborhydrid (7,71 mmol) in 30 ml DCM zugegeben. Das Reaktionsgemisch wird 18 h bei RT gerührt. Danach werden 80 ml gesättigte NaHCO₃-Lösung zugegeben und nochmals 30 min gerührt. Dann wird dreimal mit Dichlormethan extrahiert, über MgSO₄ getrocknet und am Vakuum konzentriert. Eine weitere Aufreinigung des erhaltenen orangen Öls erfolgt nicht; es wird direkt weiter eingesetzt.

### Schritt 2:

In einem 30 ml Druckgefäß wird das synthetisierte Produkt aus Schritt 1 unter Schutzgasatmosphäre in 4 ml THF gelöst und anschließend 16 ml NaOMe-Lösung (0,57 M in MeOH) zugegeben. Das Gefäß wird druckdicht verschlossen und in einer Mikrowelle 3 h bei 150 °C gerührt. Der Druck steigt dabei kontinuierlich auf 27 bar an. Nachdem der Druck konstant bleibt wird auf Raumtemperatur abgekühlt, das Reaktionsgemisch in 60 ml H₂O gegeben, dann dreimal mit je 30 ml Ether extrahiert, über MgSO₄ getrocknet und am Vakuum konzentriert. Die Aufreinigung erfolgt in einer kurzen Kieselgel-Säule (pures Ethylacetat, R_{f} = 0.04). Das Produkt ist ein farbloser Feststoff. Ausbeute 58 %.
¹H NMR(DCM-d₂, 400.13 MHz): δ 10.70 (bs, 1H), 8.39 (m, 2H), 7.52 (m, 1H), 7.48 (m, 2H), 7.09-7.11 (m, 1H), 7.06 (m, 2H), 6.97 (m, 2H), 6.90 (m, 2H), 6.44 (m, 1H), 3.98 (s, 2H), 2.82-2.86 (s, 8H).
¹³C NMR(DCM-d₂, 100.13 MHz): δ 161.49, 149.35, 136.72, 135.93, 128.55, 124.89, 123.58, 123.06, 121.82, 121.54, 119.97, 119.30, 101.55, 58.04, 54.62, 36.00.

### Beispiel 2: Synthese von [Cu(InPEA)][BF₄]

### (nicht erfindungsgemäß)

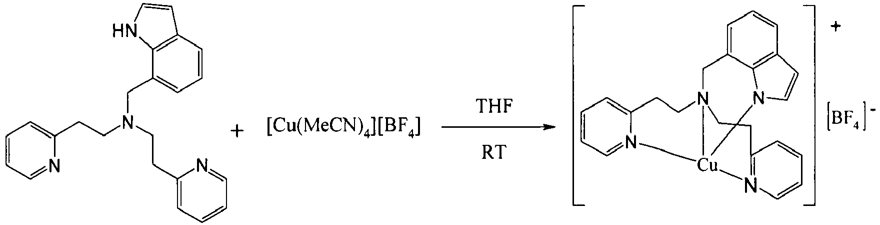

204,0 mg (0,57 mmol) InPEA aus Beispiel 1 und 180,0 mg (0,57 mmol) [Cu(MeCN)₄][BF₄] werden in einem Schlenkkolben inertisiert, in 30 ml THF gelöst und 2 h bei Raumtemperatur gerührt. Es entsteht eine gelb-braune Suspension. Diese wird filtriert und Diethylether eindiffundieren gelassen. Dabei entstehen gelbe Kristalle.
¹H NMR(DCM-d₂, 400.13 MHz): δ 9.33 (bs, 1H), 8.17 (m, 2H), 7.73 (m, 2H), 7.34 (m, 1H), 7.32 (m, 1H), 7.24 (m, 2H), 7.17-7.21 (m, 2H), 7.00 (m, 1H), 7.86 (m, 1H), 6.50 (m, 1H), 4.01 (s, 2H), 3.05-3.27 (m, 8H).
¹³C{¹H} NMR(DCM-d₂, 100.13 MHz): δ 160.67, 150.44, 146.34, 139.34, 130.01, 127.17, 125.92, 123.51, 123.33, 121.39, 120.56, 120.18, 104.64, 56.51, 54.47, 36.22.

Das [Cu(InPEA)][BF₄] Salz kann z.B. mit Kalium-tert.-Butanolat (KOtBu) deprotoniert werden. Das Produkt [Cu(InPEA)], also der neutrale Komplex, kann anhand des ¹H-NMR Spektrums eindeutig nachgewiesen werden.

### Beispiel 3: Synthese von [Cu(InPEA)]

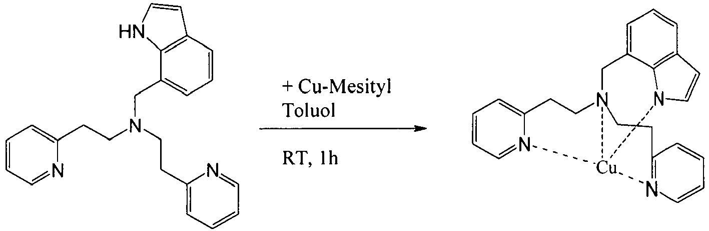

191,1 mg (0,54 mmol) InPEA aus Beispiel 1 und 98,0 mg (0,54 mmol) Cu-Mesityl werden in einer Glovebox in 4 ml Toluol gelöst und 1 h bei Raumtemperatur gerührt. Es entsteht eine orange Lösung. Diese wird filtriert und das Lösemittel am Vakuum entfernt. Der reine Komplex wird durch Sublimation bei 1 · 10⁻² mbar und 180 °C erhalten.
¹H NMR(DCM-d₂, 400.13 MHz): δ 8.40 (m, 2H), 7.60 (m, 2H), 7.49 (m, 1H), 7.42-7.45 (m, 1H), 7.13 (m, 2H), 7.06 (m, 2H), 6.77-6.82 (m, 2H), 6.44 (m, 1H), 4.10-4.34 (m, 2H), 2.68-2.99 (m, 8H).

### Beispiel 4: Synthese des Liganden InPA (N-((1H-indol-7-yl)methyl)-1-(pyridin-2-yl)-N-(pyridin-2-ylmethyl)methanamin)

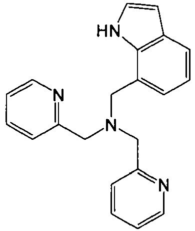

### Schritt 1:

In einem 250 ml Schlenkkolben werden 1,02 g Di(2-picolyl)amin (CAS: 1539-42-0) (5,14 mmol) und 1,26 g N-Boc-indol-7-carbaldehyd (CAS: 597544-14-4) (5,14 mmol) inertisiert und dann in 30 ml Dichlormethan gelöst. Anschließend wird eine inerte Suspension aus 1,52 g Na-triacetoxyborhydrid (7,19 mmol) in 40 ml Dichlormethan zugegeben. Das Reaktionsgemisch wird 18 h bei Raumtemperatur gerührt. Danach werden 70 ml gesättigte NaHCO₃-Lösung zugegeben und nochmals 30 min gerührt. Dann wird dreimal mit Dichlormethan extrahiert, über MgSO₄ getrocknet und am Vakuum konzentriert. Eine weitere Aufreinigung des erhaltenen orangen Öls erfolgt nicht. Ausbeute 99%.
¹H NMR(DCM-d₂, 400.13 MHz): δ 8.57 (m, 2H), 7.58-7.67 (m, 4H), 7.48-7.53 (m, 3H), 7.27 (m, 1H), 7.11 (m, 2H), 6.63 (m, 1H), 4.39 (s, 2H), 3.85 (s, 4H), 1.65 (s, 9H).
¹³C NMR(DCM-d₂, 100.13 MHz): δ 159.76, 149.98, 149.27, 136.28, 134.43, 132.55, 128.75, 127.42, 126.45, 123.56, 123.36, 122.11, 120.45, 107.47, 83.67, 58.99, 58.92, 28.35.

### Schritt 2:

In einem 30 ml Druckgefäß werden unter Schutzgasatmosphäre 2,19 g Boc-InPA (5,11 mmol) aus Schritt 1 in 8 ml THF gelöst und anschließend 12 ml NaOMe-Lösung (0,57 M in MeOH) zugegeben. Das Gefäß wird druckdicht verschlossen und in einer Mikrowelle 2 h bei 130 °C gerührt. Der Druck steigt dabei kontinuierlich auf 27 bar an. Nachdem der Druck konstant bleibt, wird auf Raumtemperatur abgekühlt, das Reaktionsgemisch in 60 ml H₂O gegeben, dann dreimal mit je 30 ml Ether extrahiert, über MgSO₄ getrocknet und am Vakuum konzentriert. Die Aufreinigung erfolgt mit einer Kieselgel-Säule (pures Ethylacetat, R_{f} = 0.28). Das Produkt ist ein farbloser Feststoff. Ausbeute 63%.
¹H NMR(DCM-d₂, 400.13 MHz): δ 12.55 (bs, 1H), 8.66 (m, 2H), 7.67 (m, 1H), 7.59 (m, 2H), 7.55-7.57 (m, 1H), 7.29 (m, 2H), 7.08-7.18 (m, 4H), 6.63-6.67 (m, 1H), 4.01 (s, 2H), 3.95 (s, 4H).
¹³C NMR(DCM-d₂, 100.13 MHz): δ 160.08, 149.40, 136.98, 136.41, 128.69, 124.86, 123.73, 123.15, 122.52, 121.97, 120.26, 119.26, 101.57, 60.11, 57.65.
Elementaranalyse: berechnet (%) für C₂₁H₂₀N₄ (328.41 g/mol): C, 76.80; H, 6.14; N, 17.06; gefunden: C, 76.44; H, 6.09; N, 17.02.

### Beispiel 5: Synthese des Liganden PBPA (N-(2-(1H-pyrrol-2-yl)benzyl)-1-(pyridin-2-yl)-N-(pyridin-2-ylmethyl)methanamin)

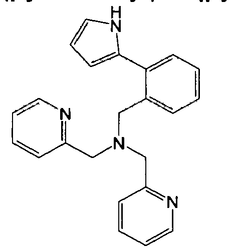

### Schritt 1:

In einem 250 ml Schlenkkolben werden 868,1 mg Di(2-picolyl)amin (4,36 mmol) und 1,182 g tert-Butyl-2-(2-formylphenyl)-1H-pyrrol-1-carboxylat (CAS: 445262-65-7) (4,36 mmol) inertisiert und dann in 20 ml Dichlormethan gelöst. Anschließend wird eine inerte Suspension aus 1,293 g Na-triacetoxyborhydrid (6,10 mmol) in 30 ml Dichlormethan zugegeben. Das Reaktionsgemisch wird 18 h bei Raumtemperatur gerührt. Danach werden 60 ml gesättigte NaHCO₃-Lösung zugegeben und nochmals 30 min gerührt. Dann wird dreimal mit Dichlormethan extrahiert, über MgSO₄ getrocknet und am Vakuum konzentriert. Eine weitere Aufreinigung des erhaltenen orangen Öls erfolgt nicht. Ausbeute 95%.
¹H NMR(DCM-d₂, 250.13 MHz): δ 8.51 (m, 2H), 7.94 (m, 1H), 7.58-7.71 (m, 5H), 7.46 (m, 1H), 7.25-7.28 (m, 1H), 7.07-7.17 (m, 3H), 6.32 (m, 1H), 6.15 (m, 1H), 3.52-3.85 (m, 6H), 1.20 (s, 9H).
¹³C NMR(DCM-d₂, 62.90 MHz): δ 160.38, 149.47, 149.37, 139.75, 136.70, 135.28, 133.21, 131.05, 128.56, 128.40, 126.52, 122.99, 122.29, 121.94, 114.73, 110.95, 83.60, 60.77, 56.21, 27.70.

### Schritt 2:

In einem 30 ml Druckgefäß werden unter Schutzgasatmosphäre 1,2 g Boc-PBPA (2,64 mmol) aus Schritt 1 in 8 ml THF gelöst und anschließend 12 ml NaOMe-Lösung (0,57 M in MeOH) zugegeben. Das Gefäß wird druckdicht verschlossen und in einer Mikrowelle 3 h bei 150 °C gerührt. Der Druck steigt dabei kontinuierlich auf 23 bar an. Danach wird auf Raumtemperatur abgekühlt, das Reaktionsgemisch in 60 ml H₂O gegeben, dann dreimal mit je 30 ml Ether extrahiert, über MgSO₄ getrocknet und am Vakuum konzentriert. Die Aufreinigung erfolgt über eine Kieselgel-Säule (pures Ethylacetat, R_{f} = 0.28). Das Produkt ist ein farbloses Öl. Ausbeute 71%.
¹H NMR(DCM-d₂, 400.13 MHz): δ 12.89 (bs, 1H), 8.59 (m, 2H), 7.66 (m, 1H), 7.55 (m, 2H), 7.39 (m, 1H), 7.33 (m, 1H), 7.22 (m, 2H), 7.17 (m, 1H), 7.10-7.14 (m, 2H), 6.99-7.02 (m, 1H), 6.46-6.49 (m, 1H), 6.27-6.30 (m, 1H), 3.93 (s, 2H), 3.92 (s, 4H).
¹³C NMR(DCM-d₂, 100.13 MHz): δ 158.81, 149.84, 136.61, 135.45, 134.04, 132.81, 132.66, 129.07, 128.83, 126.02, 124.19, 122.54, 120.20, 109.05, 107.88, 61.06, 60.30.

### Beispiel 6: Synthese des Liganden OPEA (2-((bis(2-(pyridin-2-yl)ethyl)amino)methyl)phenol)

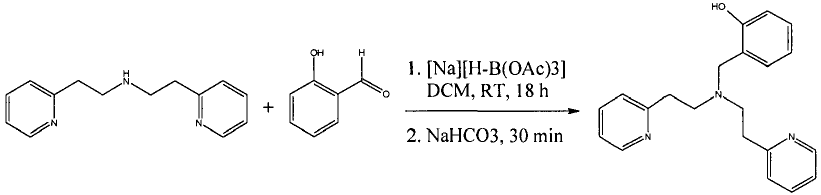

In einem 250 ml Schlenkkolben werden 2,40 g Bis(2-(pyridin-2-yl)ethyl)amin (10 mmol) und 1,29 g Salicylaldehyd (10 mmol) inertisiert und dann in 80 ml Dichlormethan gelöst. Anschließend wird eine inerte Suspension aus 3,13 g Na-triacetoxyborhydrid (15 mmol) in 80 ml Dichlormethan zugegeben. Das Reaktionsgemisch wird 18 h bei Raumtemperatur gerührt. Danach werden 160 ml gesättigte NaHCO₃-Lösung zugegeben und nochmals 30 min gerührt. Dann wird dreimal mit Dichlormethan extrahiert, über MgSO₄ getrocknet und am Vakuum konzentriert. Das erhaltene gelbe Öl wird über eine 7 cm Kieselgel-Säule gereinigt (pures Ethylacetat, R_{f} = 0.08). Ausbeute 84%.
¹H NMR(DCM-d₂, 400.13 MHz): δ 10.21 (bs, 1H), 8.53 (m, 2H), 7.54 (m, 2H), 7.16 (m, 1H), 7.05-7.11 (m, 4H), 7.03 (m, 1H), 6.76-6.83 (m, 2H), 3.87 (s, 2H), 2.97-3.10 (s, 8H).
¹³C NMR(DCM-d₂, 100.13 MHz): δ 160.00, 158.45, 149.62, 136.65, 129.19, 128.99, 123. 58, 122.78, 121.67, 119. 31, 116. 33, 58.04, 53.34, 35.09.

### Beispiel 7: Synthese von [Cu(OPEA)][BF₄]

### (nicht erfindungsgemäß)

333,3 mg (1 mmol) OPEA aus Beispiel 6 und 314,6 mg (1 mmol) [Cu(MeCN)₄][BF₄] werden in einem Schlenkkolben inertisiert, in 40 ml THF gelöst und 2 h bei Raumtemperatur gerührt. Es entsteht eine gelbe Lösung. Diese wird filtriert und mit n-Hexan überschichtet. Dabei entstehen gelbe Kristalle. Ausbeute: 91%.
¹H NMR(DCM-d₂, 400.13 MHz): δ 8.80 (m, 2H), 8.46 (bs, 1H), 7.69 (m, 2H), 7.28 (m, 2H), 7.18 (m, 2H), 6.96 (m, 1H), 6.89 (m, 1H), 6.85 (m, 1H). 6.61 (m, 1H), 3.66 (s, 2H), 2.80-3.18 (m, 8H). ¹³C{¹H} NMR(DCM-d₂, 100.13 MHz): δ 160.18, 155.01, 151.65, 138.52, 132.48, 130.28, 125.18, 123.67, 122.34, 120.51, 116.38, 57.22, 55.98, 36.10.
Elementaranalyse berechnet (%) für C₂₁H₂₃BCuF₄N₃O (483.78 g/mol): C, 52.14; H, 4.79; N, 8.69. Gefunden: C, 52.05; H, 4.64; N, 8.67.

### Beispiel: Herstellung der OLEDs

### 1) Vakuum-prozessierte Devices:

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Glasplättchen, mit strukturiertem ITO (50 nm, Indium Zinn Oxid) bilden die Substrate, auf welche die OLEDs aufgebracht werden. Die OLEDs haben prinzipiell folgenden Schichtaufbau: Substrat / Lochtransportschicht 1 (HTL1) bestehend aus HTM dotiert mit 3 % NDP-9 (kommerziell erhältlich von der Fa. Novaled), 20 nm / Lochtransportschicht 2 (HTL2) / optionale Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet.

Zunächst werden vakuumprozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie M1:M2:[Cu(InPEA)] (55%:35%:10%) bedeutet hierbei, dass das Material M1 in einem Volumenanteil von 55%, M2 in einem Anteil von 35% und [Cu(InPEA)] in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 4 gezeigt.

Die OLEDs werden standardmäßig charakterisiert. Hierfür werden die Elektrolumineszenzspektren, die Stromeffizienz (gemessen in cd/A) und die Spannung (gemessen bei 1000 cd/m² in V) bestimmt aus Strom-Spannungs-Helligkeits-Kennlinien (IUL-Kennlinien).

### Verwendung von erfindungsgemäßen Verbindungen als Emittermaterialien in OLEDs

Die erfindungsgemäßen Verbindungen lassen sich unter anderem als Emittermaterialien in der Emissionsschicht in OLEDs einsetzen.

**Tabelle 1: Aufbau der OLED**

| **Bsp.** | **HTL2 Dicke** | **EBL Dicke** | **EML Dicke** | **HBL Dicke** | **ETL Dicke** |
|---|---|---|---|---|---|
| **Rote OLEDs** | | | | | |
| D1 | HTM 230 nm | --- | M1:M2:[Cu(InPEA)] (65%:30%:5%) 35 nm | --- | ETM1:ETM2 (50%:50%) 40 nm |
| D2 | HTM 230 nm | --- | M1:M2:[Cu(InPEA)] (60%:30%:10%) 35 nm | --- | ETM 1: ETM2 (50%:50%) 40 nm |
| D3 | HTM 220 nm | 10 nm | M1:M2:[Cu(InPEA)] (65%:30%:5%) 35 nm | --- | ETM1:ETM2 (50%:50%) 40 nm |

**Tabelle 2: Ergebnisse der Vakuum-prozessierten OLEDs**

| **Bsp.** | **EQE (%) 1000 cd/m²** | **Spannung (V) 1000 cd/m²** | **CIE x/y 1000 cd/m²** |
|---|---|---|---|
| D1 | 9.4 | 3.9 | 0.51/0.40 |
| D2 | 11.7 | 3.7 | 0.51/0.41 |
| D3 | 17.2 | 4.2 | 0.49/0.43 |

### 2) Lösungs-prozessierte Devices aus löslichen Funktionsmaterialien

Die erfindungsgemäßen Komplexe können auch aus Lösung verarbeitet werden und führen dort zu prozesstechnisch einfacheren OLEDs im Vergleich zu den vakuumprozessierten OLEDs, mit dennoch guten Eigenschaften. Die Herstellung solcher Bauteile lehnt sich an die Herstellung polymerer Leuchtdioden (PLEDs) an, die in der Literatur bereits vielfach beschrieben ist (z.B. in der WO 2004/037887). Der Aufbau setzt sich aus Substrat / ITO / PEDOT (80 nm) / Interlayer (80 nm) / Emissionsschicht (80 nm) / Kathode zusammen. Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum als Pufferschicht eine 80 nm Schicht PEDOT (PEDOT ist ein Polythiophen-Derivat (Baytron P VAI 4083sp.) von H. C. Starck, Goslar, das als wässrige Dispersion geliefert wird) durch Spin-Coating aufgebracht. Die benötigte Spinrate hängt vom Verdünnungsgrad und der spezifischen Spin-Coater-Geometrie ab (typisch für 80 nm: 4500 rpm). Um Restwasser aus der Schicht zu entfernen, werden die Substrate für 10 Minuten bei 180 °C auf einer Heizplatte ausgeheizt. Die verwendete Interlayer dient der Lochinjektion, in diesem Fall wird HIL-012 von Merck verwendet. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die erfindungsgemäßen Emitter zusammen mit den Matrixmaterialien in Toluol oder THF gelöst. Der typische Feststoffgehalt solcher Lösungen liegt zwischen 16 und 25 g/L, wenn, wie hier, die für eine Device typische Schichtdicke von 80 nm mittels Spincoating erzielt werden soll. Die lösungsprozessierten Devices enthalten eine Emissionsschicht aus (Polystyrol):M3:M4:Emitter (25%:25%:40%:10%). Die Emissionsschicht wird in einer Inertgasatmosphäre, im vorliegenden Fall Argon, aufgeschleudert und 30 min bei 130 °C ausgeheizt. Zuletzt wird eine Kathode aus Barium (5 nm) und dann Aluminium (100 nm) (hochreine Metalle von Aldrich, besonders Barium 99.99 % (Best-Nr. 474711); Aufdampfanlagen von Lesker o.a., typischer Aufdampfdruck 5 x 10⁻⁶ mbar) aufgedampft. Optional kann zunächst eine Lockblockierschicht und dann eine Eletronentransportschicht und dann erst die Kathode (z.B. Al oder LiF/Al) im Vakuum aufgedampft werden. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 3 fasst die erhaltenen Daten zusammen.

**Tabelle 3: Ergebnisse mit aus Lösung prozessierten Materialien**

| **Bsp.** | **Emitter** | **EQE (%) 1000 cd/m²** | **Spannung (V) 1000 cd/m²** | **CIE x/y 1000 cd/m²** |
|---|---|---|---|---|
| Sol-D1 | [Cu(InPEA)] | 8.8 | 4.9 | 0.51/0.40 |
| Sol-D2* | [Cu(InPEA)][BF₄] | 6.7 | 5.7 | 0.52/0.28 |
| Sol-D3* | [Cu(OPEA)][BF₄] | 7.0 | 5.5 | 0.55/0.38 |

| | | | | |
|---|---|---|---|---|
| * nicht erfindungsgemäß | | | | |

**Tabelle 4: Strukturformeln der verwendeten Materialien**

| | |
|---|---|
| 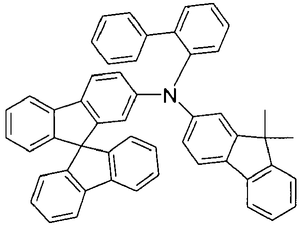 | 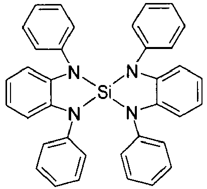 |
| HTM | EBM |
| 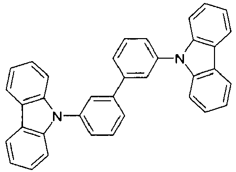 | 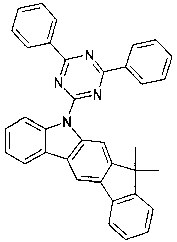 |
| M1 | M2 |
| 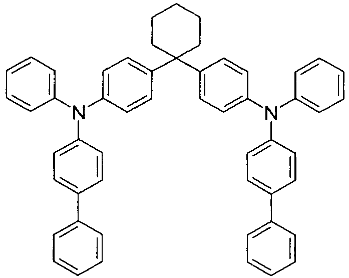 | 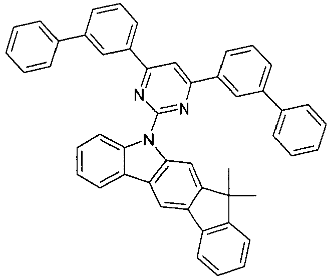 |
| M3 | M4 |
| 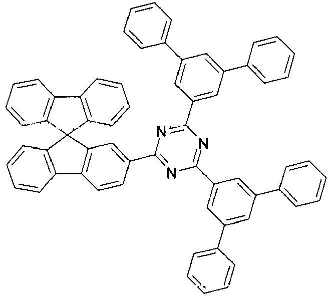 | 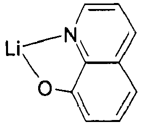 |
| ETM1 | ETM2 |

## Patentansprüche

1. Elektrisch neutrale Verbindung gemäß Formel (1), 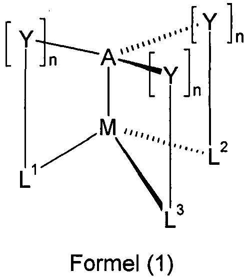 wobei für die verwendeten Symbole und Indizes gilt:
M ist Cu(I);
A ist ausgewählt aus N oder P;
Y ist bei jedem Auftreten gleich oder verschieden eine bivalente Gruppe, ausgewählt aus CR₂, 1,2-Phenylen oder eine orthoverknüpfte Heteroarylengruppe mit 5 oder 6 aromatischen Ringatomen, wobei diese Gruppen jeweils mit einem oder mehreren Resten R substituiert sein kann;
L¹, L², L³ ist gleich oder verschieden bei jedem Auftreten eine Heteroarylgruppe mit 5 bis 14 aromatischen Ringatomen, die mit einem oder mehreren Resten R substituiert sein kann und die ein Stickstoff-, Schwefel- oder Sauerstoffatom enthält, welches an M koordinieren kann, oder eine Aryl- oder Heteroarylgruppe mit 5 bis 14 aromatischen Ringsatomen, die mit einem oder mehreren Resten R substituiert sein kann und die ein exocyclisches Donoratom, ausgewählt aus N, O, S oder P, aufweist, welches an M koordiniert und welches durch einen oder mehrere Reste R substituiert sein kann; dabei können die Teilliganden L¹, L² und L³ auch über Reste R, die miteinander verbrückt sind, miteinander verbunden sein;
n ist bei jedem Auftreten gleich oder verschieden 0, 1 oder 2;
R ist bei jedem Auftreten gleich oder verschieden H, D, F, Cl, Br, I, N(R¹)₂, CN, NO₂ OR¹, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenyl- oder Alkinylgruppe mit 2 bis 40 C-Atomen, die jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=NR¹, P(=O)(R¹), SO, SO₂, NR¹, O, S oder CONR¹ ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy-, Heteroaryloxy-, Aralkyl- oder Heteroaralkylgruppe mit 5 bis 60 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei oder mehrere Substituenten R auch miteinander ein mono- oder polycyclisches, aliphatisches, aromatisches, heteroaromatisches und/oder benzoannelliertes Ringsystem bilden;
R¹ ist bei jedem Auftreten gleich oder verschieden H, D, F, Cl, Br, I, N(R²)₂, CN, NO₂ OH, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenyl- oder Alkinylgruppe mit 2 bis 40 C-Atomen, die jeweils mit einem oder mehreren Resten R² substituiert sein kann, wobei eine oder mehrere nicht benachbarte CH₂-Gruppen durch R²C=CR², C=C, Si(R²)₂, C=O, C=S, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy-, Heteroaryloxy-, Aralkyl- oder Heteroaralkylgruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann; dabei können zwei oder mehrere Substituenten R¹ auch miteinander ein mono- oder polycyclisches, aliphatisches, aromatisches, heteroaromatisches und/oder benzoannelliertes Ringsystem bilden;
R² ist bei jedem Auftreten gleich oder verschieden H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R² auch miteinander ein mono- oder polycyclisches aliphatisches, aromatisches, heteroaromatisches und/oder benzoannelliertes Ringsystem bilden.

2. Verbindung nach Anspruch 1, **dadurch gekennzeichnet, dass** der Cyclus, der aus A, Y, M und L¹ bzw. L² bzw. L³ gebildet wird, 5, 6, 7, 8 oder 9 Ringatome, bevorzugt 5, 6, 7 oder 8 Ringatome und besonders bevorzugt 5, 6 oder 7 Ringatome.

3. Verbindung nach Anspruch 1 oder 2, **dadurch gekennzeichnet, dass** L¹ bzw. L² bzw. L³ 5 bis 13 aromatische Ringatome aufweist, bevorzugt 5 bis 10 aromatische Ringatome, wobei die Aryl- bzw. Heteroarylgruppen durch einen oder mehrere Reste R substituiert sein können.

4. Verbindung nach einem oder mehreren der Ansprüche 1 bis 3, **dadurch gekennzeichnet, dass** L¹, L² und L³ gleich oder verschieden bei jedem Auftreten ausgewählt sind aus den Gruppen der Formeln (2) bis (41): 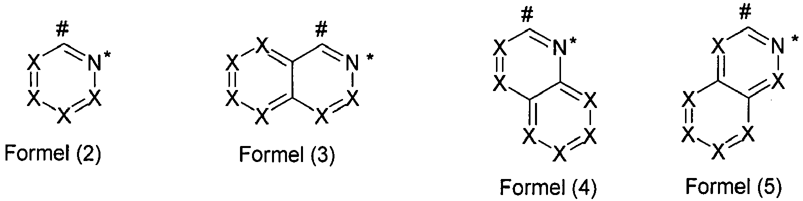 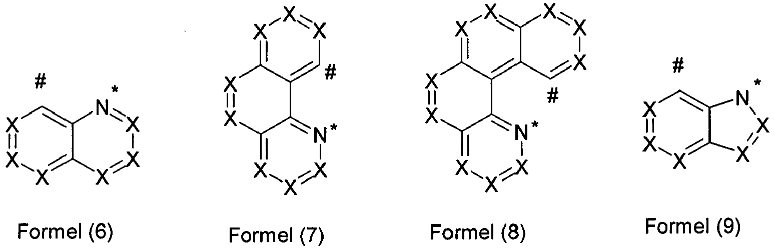 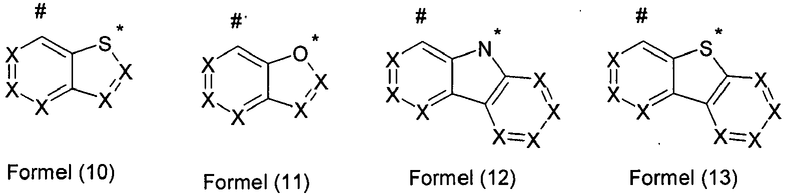 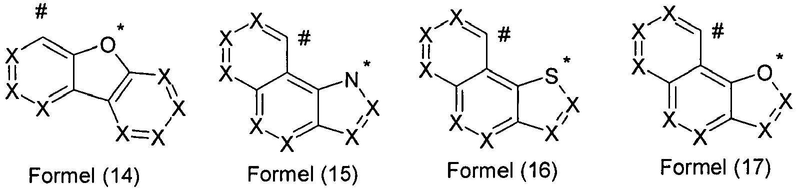 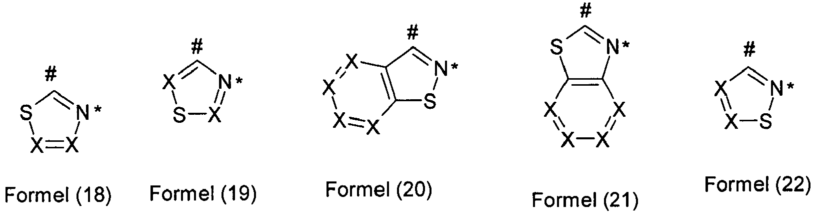 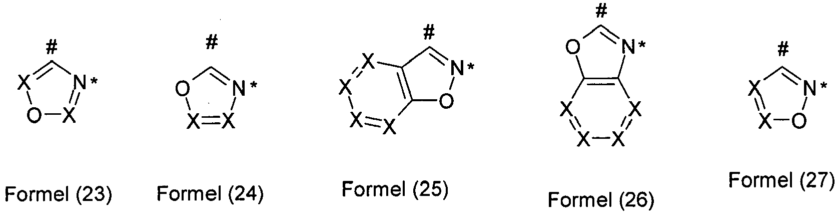 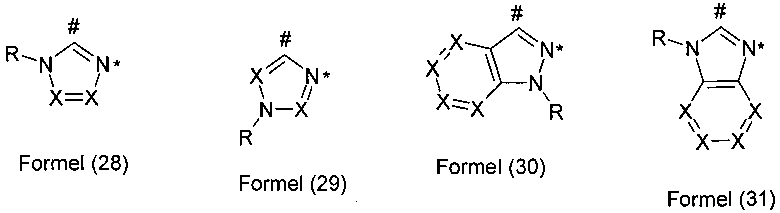 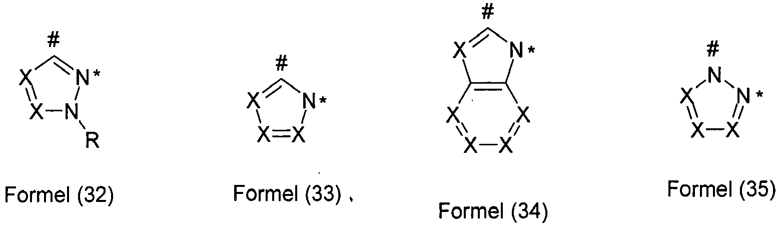 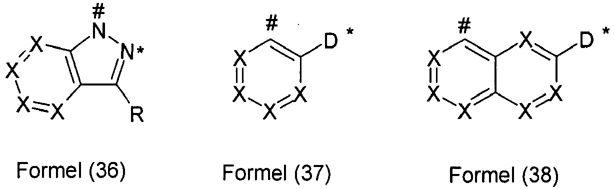 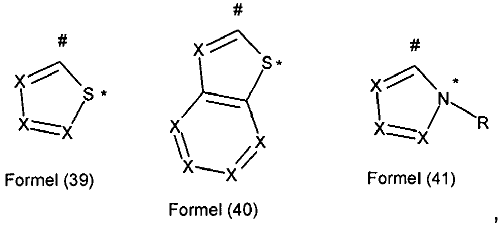 wobei R dieselbe Bedeutung hat, wie in Anspruch 1 beschrieben, die Gruppen an das Metall M über die durch * gekennzeichnete Position koordinieren, die durch # gekennzeichnete Position die Position angibt, an der der Teilligand L¹ bzw. L² bzw. L³ an Y bzw. an A gebunden ist, und weiterhin gilt:
X steht bei jedem Auftreten gleich oder verschieden für CR oder N;
D steht bei jedem Auftreten gleich oder verschieden für OH, O- SH, S⁻, NR², NR-, PR⁻,PR₂, OR, SR, COO-, -C(=O)R, -CR(=NR) oder -N(=CR₂).

5. Verbindung nach einem oder mehreren der Ansprüche 1 bis 4, **dadurch gekennzeichnet, dass** Y gleich oder verschieden bei jedem Auftreten eine bivalente Gruppe ist, ausgewählt aus CR₂ oder O, besonders bevorzugt CR₂, mit der Maßgabe, dass Y nicht O ist, wenn A = N ist.

6. Verbindung nach einem oder mehreren der Ansprüche 1 bis 5, **dadurch gekennzeichnet, dass** für die verwendeten Symbole und Indizes gilt:
L¹, L², L³ ist gleich oder verschieden bei jedem Auftreten ausgewählt aus den oben genannten Gruppen gemäß den Formeln (2) bis (41), wobei maximal drei Symbole X in jeder Gruppe für N stehen;
Y ist gleich oder verschieden bei jedem Auftreten eine bivalente Gruppe, ausgewählt aus CR₂ oder O;
n ist gleich oder verschieden bei jedem Auftreten 0, 1 oder 2;
und die weiteren verwendeten Symbole und Indizes die in Anspruch 1 und 4 genannten Bedeutungen aufweisen.

7. Verbindung nach einem oder mehreren der Ansprüche 1 bis 6, **dadurch gekennzeichnet, dass** mindestens zwei der Teilliganden L¹, L² und L³ gleich sind und gleich substituiert sind.

8. Verfahren zur Herstellung einer Verbindung nach einem oder mehreren der Ansprüche 1 bis 7, **gekennzeichnet durch** die Umsetzung eines Metallsalzes bzw. Metallkomplexes des Metalls M mit dem entsprechenden freien Liganden, gegebenenfalls in deprotonierter Form, optional gefolgt von einem Deprotonierungsschritt.

9. Formulierung, enthaltend mindestens eine Verbindung nach einem oder mehreren der Ansprüche 1 bis 7 und mindestens eine weitere Verbindung, insbesondere ein Lösemittel.

10. Verwendung einer Verbindung nach einem oder mehreren der Ansprüche 1 bis 7 in einer elektronischen Vorrichtung, zur Erzeugung von Singulett-Sauerstoff, in der Photokatalyse oder in Sauerstoffsensoren.

11. Elektronische Vorrichtung, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden, enthaltend in mindestens einer Schicht mindestens eine Verbindung nach einem oder mehreren der Ansprüche 1 bis 7.

12. Elektronische Vorrichtung nach Anspruch 11, wobei es sich um eine organische Elektrolumineszenzvorrichtung handelt, **dadurch gekennzeichnet, dass** die Verbindung nach einem oder mehreren der Ansprüche 1 bis 7 in einer emittierenden Schicht als Emitter oder als Matrix oder in einer Lochblockierschicht oder in einer Elektronentransportschicht enthalten ist.

## Claims

1. Electrically neutral compound of the formula (1), 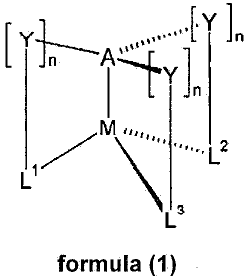 where the following applies to the symbols used:
M is Cu(I);
A is selected from N or P;
Y is on each occurrence, identically or differently, a divalent group selected from CR₂, 1,2-phenylene or an ortho-linked heteroarylene group having 5 or 6 aromatic ring atoms, where these groups may each be substituted by one or more radicals R;
L¹, L², L³ are, identically or differently on each occurrence, a heteroaryl group having 5 to 14 aromatic ring atoms, which may be substituted by one or more radicals R and which contains a nitrogen, sulfur or oxygen atom, which may be coordinated to M, or an aryl or heteroaryl group having 5 to 14 aromatic ring atoms, which may be substituted by one or more radicals R and which contains an exo-cyclic donor atom selected from N, O, S or P, which may be coordinated to M and which may be substituted by one or more radicals R; the part-ligands L¹, L² and L³ may also be connected to one another via radicals R which are bridged to one another;
n is on each occurrence, identically or differently, 0, 1 or 2;
R is on each occurrence, identically or differently, H, D, F, Cl, Br, I, N(R¹)₂, CN, NO₂ OR¹, Si(R¹)₃, B(OR¹)₂, C(=O)R¹, P(=O)(R¹)₂, S(=O)R¹, S(=O)₂R¹, OSO₂R¹, a straight-chain alkyl, alkoxy or thioalkoxy group having 1 to 40 C atoms or a branched or cyclic alkyl, alkoxy or thioalkoxy group having 3 to 40 C atoms or an alkenyl or alkynyl group having 2 to 40 C atoms, which may in each case be substituted by one or more radicals R¹, where one or more non-adjacent CH₂ groups may be replaced by R¹C=CR¹, C=C, Si(R¹)₂, C=O, C=S, C=NR¹, P(=O)(R¹), SO, SO₂, NR¹, O, S or CONR¹ and where one or more H atoms may be replaced by F, Cl, Br, I, CN or NO₂ or an aromatic or heteroaromatic ring system having 5 to 60 aromatic ring atoms, which may in each case be substituted by one or more radicals R¹, or an aryloxy, heferoaryloxy, aralkyl or heteroaralkyl group having 5 to 60 aromatic ring atoms, which may be substituted by one or more radicals R¹, or a diarylamino group, diheteroarylamino group or arylheteroarylamino group having 10 to 40 aromatic ring atoms, which may be substituted by one or more radicals R¹; two or more substituents R may also form a mono- or polycyclic, aliphatic, aromatic, heteroaromatic and/or benzo-fused ring system with one another;
R¹ is on each occurrence, identically or differently, H, D, F, Cl, Br, I, N(R²)₂, CN, NO₂ OH, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 40 C atoms or a branched or cyclic alkyl, alkoxy or thioalkoxy group having 3 to 40 C atoms or an alkenyl or alkynyl group having 2 to 40 C atoms, which may in each case be substituted by one or more radicals R², where one or more non-adjacent CH₂ groups may be replaced by R²C=CR², C=C, Si(R²)₂, C=O, C=S, C=NR², P(=O)(R²), SO, SO₂, NR², O, S or CONR² and where one or more H atoms may be replaced by F, Cl, Br, I, CN or NO₂ or an aromatic or heteroaromatic ring system having 5 to 60 aromatic ring atoms, which may in each case be substituted by one or more radicals R², or an aryloxy, heteroaryloxy, aralkyl or heteroaralkyl group having 5 to 60 aromatic ring atoms, which may be substituted by one or more radicals R², or a diarylamino group, diheteroarylamino group or arylheteroarylamino group having 10 to 40 aromatic ring atoms, which may be substituted by one or more radicals R²; two or more substituents R¹ may also form a mono- or polycyclic, aliphatic, aromatic, heteroaromatic and/or benzo-fused ring system with one another;
R² is on each occurrence, identically or differently, H, D, F or an aliphatic, aromatic and/or heteroaromatic hydrocarbon radical having 1 to 20 C atoms, in which, in addition, one or more H atoms may be replaced by F; two or more substituents R² may also form a mono- or polycyclic, aliphatic, aromatic, heteroaromatic and/or benzo-fused ring system with one another.

2. Compound according to Claim 1, **characterised in that** the ring formed from A, Y, M and L¹ or L² or L³ has 5, 6, 7, 8 or 9 ring atoms, preferably 5, 6, 7 or 8 ring atoms and particularly preferably 5, 6 or 7 ring atoms.

3. Compound according to Claim 1 or 2, **characterised in that** L¹ or L² or L³ has 5 to 13 aromatic ring atoms, preferably 5 to 10 aromatic ring atoms, where the aryl or heteroaryl groups may be substituted by one or more radicals R.

4. Compound according to one or more of Claims 1 to 3, **characterised in that** L¹, L² and L³ are selected, identically or differently on each occurrence, from the groups of the formulae (2) to (41): 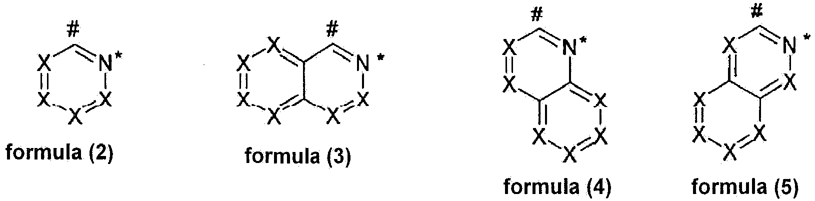 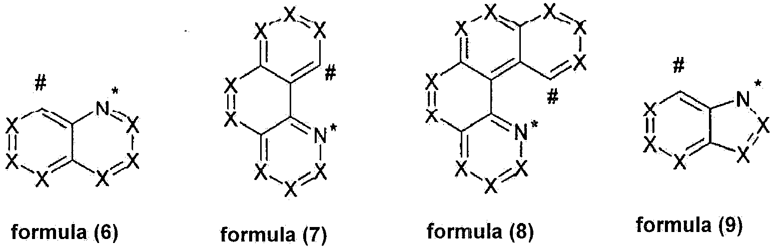 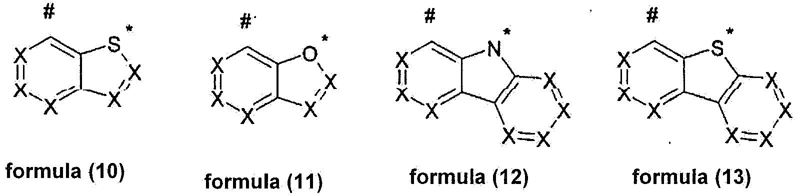 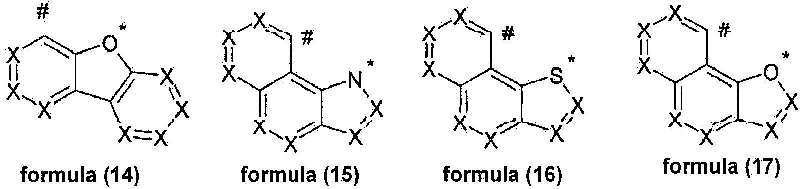 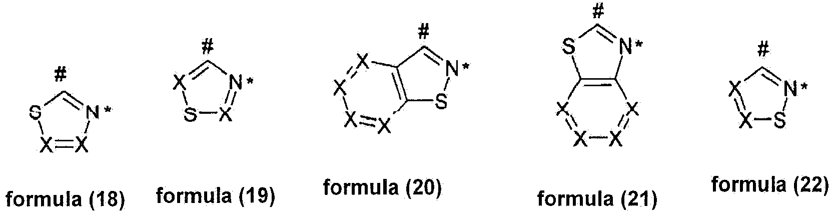 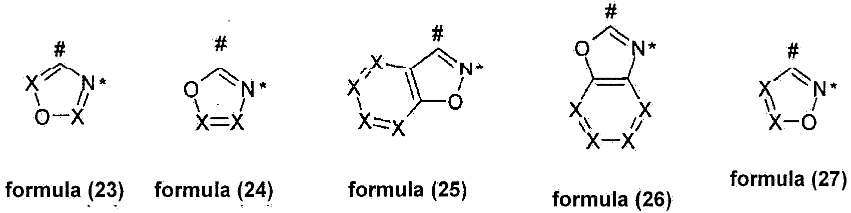 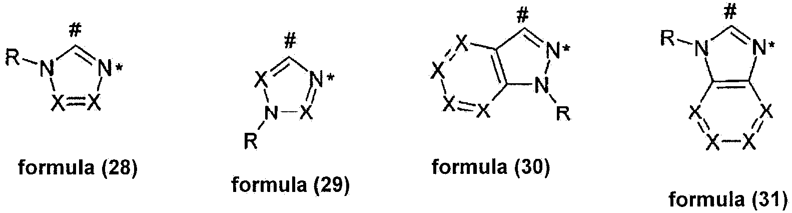 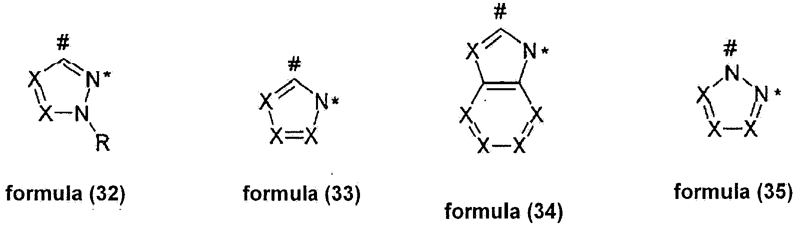 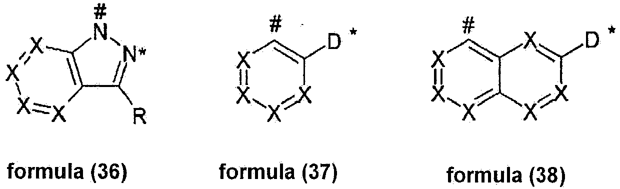 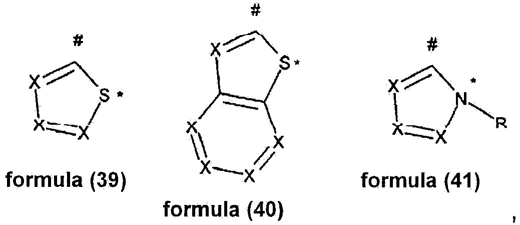 where R has the same meaning as described in Claim 1, the groups are coordinated to the metal M via the position denoted by *, the position denoted by # indicates the position at which the part-ligand L¹ or L² or L³ is bonded to Y or to A, and furthermore:
X stands on each occurrence, identically or differently, for CR or N;
D stands on each occurrence, identically or differently, for OH, O⁻, SH, S-, NR², NR-, PR-, PR₂, OR, SR, COO-, -C(=O)R, -CR(=NR) or -N(=CR₂).

5. Compound according to one or more of Claims 1 to 4, **characterised in that** Y is, identically or differently on each occurrence, a divalent group selected from CR₂ or O, particularly preferably CR₂, with the proviso that Y is not O if A = N.

6. Compound according to one or more of Claims 1 to 5, **characterised in that** the following applies to the symbols and indices used:
L¹, L², L³ are selected, identically or differently on each occurrence, from the above-mentioned groups of the formulae (2) to (41), where a maximum of three symbols X in each group stand for N;
Y is, identically or differently on each occurrence, a divalent group selected from CR₂ or O;
n is, identically or differently on each occurrence, 0, 1 or 2;
and the other symbols and indices used have the meanings given in Claims 1 and 4.

7. Compound according to one or more of Claims 1 to 6, **characterised in that** at least two of the part-ligands L¹, L² and L³ are identical and are identically substituted.

8. Process for the preparation of a compound according to one or more of Claims 1 to 7, **characterised by** the reaction of a metal salt or metal complex of the metal M with the corresponding free ligands, optionally in deprotonated form, optionally followed by a deprotonation step.

9. Formulation comprising at least one compound according to one or more of Claims 1 to 7 and at least one further compound, in particular a solvent.

10. Use of a compound according to one or more of Claims 1 to 7 in an electronic device for the generation of singlet oxygen, in photocatalysis or in oxygen sensors.

11. Electronic device, in particular selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes, containing at least one compound according to one or more of Claims 1 to 7 in at least one layer.

12. Electronic device according to Claim 11, which is an organic electroluminescent device, **characterised in that** the compound according to one or more of Claims 1 to 7 is present in an emitting layer as emitter or as matrix or in a hole-blocking layer or in an electron-transport layer.

## Revendications

1. Composé électriquement neutre de la formule (1) : 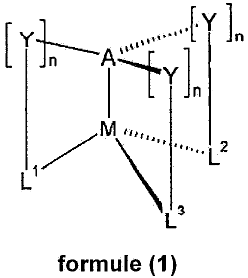 dans laquelle ce qui suit s'applique aux symboles qui sont utilisés :
M est Cu(I) ;
A est sélectionné parmi N et P ;
Y est pour chaque occurrence, de manière identique ou différente, un groupe divalent qui est sélectionné parmi CR₂, 1,2-phénylène ou un groupe hétéroarylène ortho-lié qui comporte 5 ou 6 atomes de cycle aromatique, où ces groupes peuvent chacun être substitués par un radical ou par plusieurs radicaux R ;
L¹, L², L³ sont, de manière identique ou différente pour chaque occurrence, un groupe hétéroaryle qui comporte de 5 à 14 atomes de cycle aromatique, lequel peut être substitué par un radical ou par plusieurs radicaux R et lequel contient un atome d'azote, de soufre ou d'oxygène, lequel peut être coordonné sur M, ou un groupe aryle ou hétéroaryle qui comporte 5 à 14 atomes de cycle aromatique, lequel peut être substitué par un radical ou par plusieurs radicaux R et lequel contient un atome donneur exocyclique qui est sélectionné parmi N, O, S et P, lequel peut être coordonné sur M et lequel peut être substitué par un radical ou par plusieurs radicaux R ; les ligands partiels L¹, L² et L³ peuvent également être connectés les uns aux autres via des radicaux R qui sont pontés l'un à l'autre ou les uns aux autres ;
n est pour chaque occurrence, de manière identique ou différente, 0, 1 ou 2 ;
R est pour chaque occurrence, de manière identique ou différente, H, D, F, Cl, Br, I, N(R¹)₂, CN, NO₂ OR¹, Si(R¹)₃, B(OR¹)₂, C(=O)R¹, P(=O)(R¹)₂, S(=O)R¹, S(=O)₂R¹, OSO₂R¹, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C ou un groupe alkényle ou alkynyle qui comporte de 2 à 40 atomes de C, lequel peut dans chaque cas être substitué par un radical ou par plusieurs radicaux R¹, où un ou plusieurs groupe(s) CH₂ non adjacents peut/peuvent être remplacé(s) par R¹C=CR¹, C=C, Si(R¹)₂, C=O, C=S, C=NR¹, P(=O)(R¹), SO, SO₂, NR¹, O, S ou CONR¹ et où un ou plusieurs atome(s) de H peut/peuvent être remplacé(s) par F, Cl, Br, I, CN ou NO₂ ou un système de cycle aromatique ou hétéroaromatique qui comporte de 5 à 60 atomes de cycle aromatique, lequel peut dans chaque cas être substitué par un radical ou par plusieurs radicaux R¹, ou un groupe aryloxy, hétéroaryloxy, aralkyle ou hétéroaralkyle qui comporte de 5 à 60 atomes de cycle aromatique, lequel peut être substitué par un radical ou par plusieurs radicaux R¹, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux R¹; deux substituants R ou plus peuvent également former un système de cycle aliphatique, aromatique, hétéroaromatique monocyclique ou polycyclique et/ou un système de cycle benzo-fusionné l'un avec l'autre ou les uns avec les autres;
R¹ est pour chaque occurrence, de manière identique ou différente, H, D, F, Cl, Br, I, N(R²)₂, CN, NO₂, OH, Si(R²)₃, B(OR²)₂, C(=O)R², P(=O)(R²)₂, S(=O)R², S(=O)₂R², OSO₂R², un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C ou un groupe alkényle ou alkynyle qui comporte de 2 à 40 atomes de C, lequel peut dans chaque cas être substitué par un radical ou par plusieurs radicaux R², où un ou plusieurs groupe(s) CH₂ non adjacents peut/peuvent être remplacé(s) par R²C=CR², C=C, Si(R²)₂, C=O, C=S, C=NR², P(=O)(R²), SO, SO₂, NR², O, S ou CONR² et où un ou plusieurs atome(s) de H peut/peuvent être remplacé(s) par F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R², ou un groupe aryloxy, hétéroaryloxy, aralkyle ou hétéroaralkyle qui comporte de 5 à 60 atomes de cycle aromatique, lequel peut être substitué par un radical ou par plusieurs radicaux R², ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux R²; deux substituants R¹ ou plus peuvent également former un système de cycle aliphatique, aromatique, hétéroaromatique monocyclique ou polycyclique et/ou benzo-fusionné l'un avec l'autre ou les uns avec les autres ;
R² est pour chaque occurrence, de manière identique ou différente, H, D, F ou un radical hydrocarbone aliphatique, aromatique et/ou hétéroaromatique qui comporte de 1 à 20 atome(s) de C, dans lequel, en outre, un ou plusieurs atome(s) de H peut/peuvent être remplacé(s) par F ; deux substituants R² ou plus peuvent également former un système de cycle aliphatique, aromatique, hétéroaromatique monocyclique ou polycyclique et/ou benzo-fusionné l'un avec l'autre ou les uns avec les autres.

2. Composé selon la revendication 1, **caractérisé en ce que** le cycle qui est formé à partir de A, de Y, de M et de L¹ ou de L² ou de L³ comporte 5, 6, 7, 8 ou 9 atomes de cycle, de préférence 5, 6, 7 ou 8 atomes de cycle et de façon particulièrement préférable 5, 6 ou 7 atomes de cycle.

3. Composé selon la revendication 1 ou 2, **caractérisé en ce que** L¹ ou L² ou L³ comporte de 5 à 13 atomes de cycle aromatique, de préférence de 5 à 10 atomes de cycle aromatique, où les groupes aryle ou hétéroaryle peuvent être substitués par un radical ou par plusieurs radicaux R.

4. Composé selon une ou plusieurs des revendications 1 à 3, **caractérisé en ce que** L¹, L² et L³ sont sélectionnés, de manière identique ou différente pour chaque occurrence, parmi les groupes des formules (2) à (41): 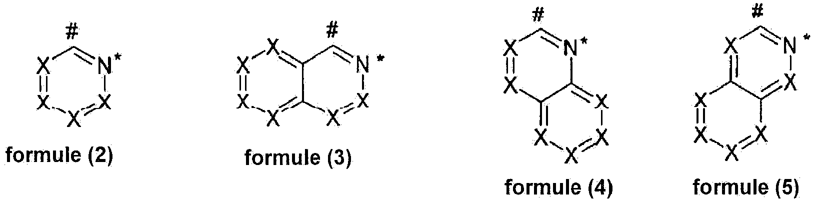 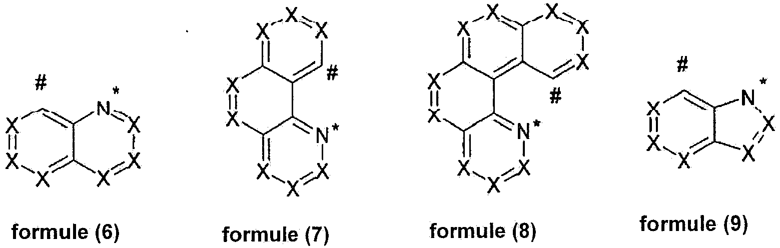 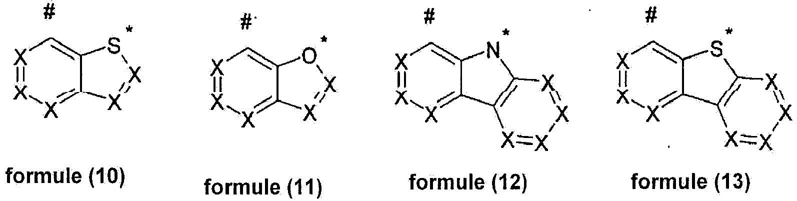 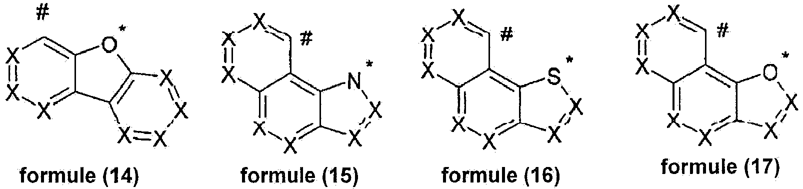 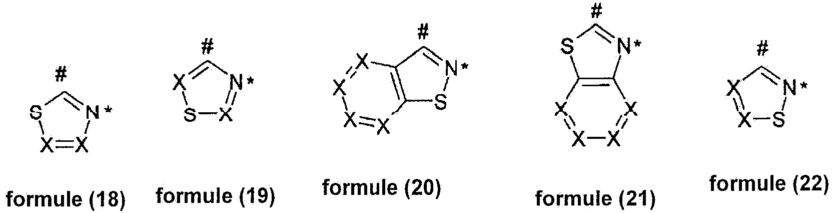 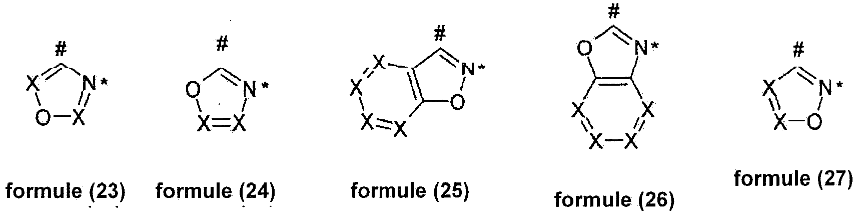 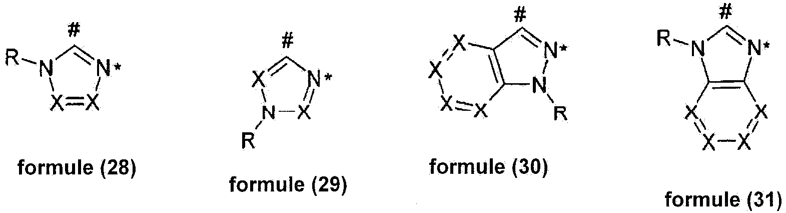 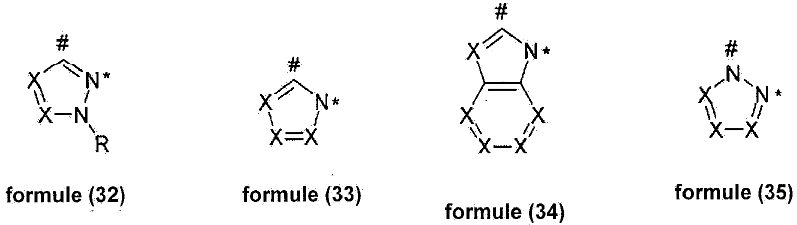 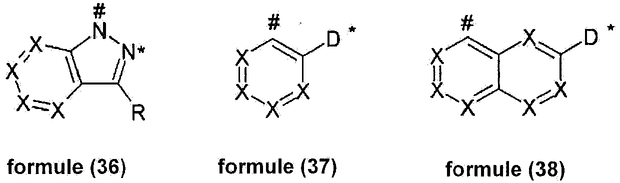 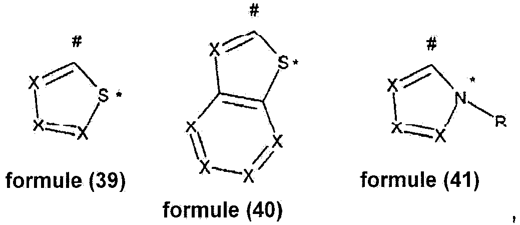 dans lesquelles R présente la même signification que décrit selon la revendication 1, les groupes sont coordonnés sur le métal M via the position qui est indiquée par*, la position qui est indiquée par # indique la position au niveau de laquelle le ligand partiel L¹ ou L² ou L³ est lié à Y ou à A, et en outre :
X représente pour chaque occurrence, de manière identique ou différente, CR ou N ;
D représente pour chaque occurrence, de manière identique ou différente, OH, O-, SH, S-, NR₂, NR-, PR-, PR₂, OR, SR, COO⁻, -C(=O)R, -CR(=NR) ou -N(=CR₂).

5. Composé selon une ou plusieurs des revendications 1 à 4, **caractérisé en ce que** Y est, de manière identique ou différente pour chaque occurrence, un groupe divalent qui est sélectionné parmi CR₂ et O, de façon particulièrement préférable CR₂, étant entendu que Y n'est pas O si A = N.

6. Composé selon une ou plusieurs des revendications 1 à 5, **caractérisé en ce que** ce qui suit s'applique aux symboles et indices qui sont utilisés :
L¹, L², L³ sont sélectionnés, de manière identique ou différente pour chaque occurrence, parmi les groupes qui ont été mentionnés ci-avant des formules (2) à (41), où un maximum de trois symboles X dans chaque groupe représentent N ;
Y est, de manière identique ou différente pour chaque occurrence, un groupe divalent qui est sélectionné parmi CR₂ et O;
n est, de manière identique ou différente pour chaque occurrence, 0, 1 ou 2 ;
et les autres symboles et indices qui sont utilisés présentent les significations qui ont été données selon les revendications 1 et 4.

7. Composé selon une ou plusieurs des revendications 1 à 6, **caractérisé en ce qu'**au moins deux des ligands partiels L¹, L² et L³ sont identiques et sont substitués de manière identique.

8. Procédé pour la préparation d'un composé selon une ou plusieurs des revendications 1 à 7, **caractérisé par** la réaction d'un sel de métal ou d'un complexe de métal du métal M avec les ligands libres correspondants, en option sous forme déprotonée, en option cette réaction étant suivie par une étape de déprotonation.

9. Formulation comprenant au moins un composé selon une ou plusieurs des revendications 1 à 7 et au moins un autre composé, en particulier un solvant.

10. Utilisation d'un composé selon une ou plusieurs des revendications 1 à 7 dans un dispositif électronique pour la génération d'oxygène singulet, pour une photocatalyse ou dans des capteurs d'oxygène.

11. Dispositif électronique, en particulier sélectionné parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les détecteurs optiques organiques, les photorécepteurs organiques, les dispositifs à extinction de champ organiques, les cellules électrochimiques à émission de lumière ou les diodes laser organiques, contenant au moins un composé selon une ou plusieurs des revendications 1 à 7 dans au moins une couche.

12. Dispositif électronique selon la revendication 11, lequel est un dispositif électroluminescent organique, **caractérisé en ce que** le composé selon une ou plusieurs des revendications 1 à 7 est présent dans une couche d'émission en tant qu'émetteur ou que matrice ou dans une couche de trous ou dans une couche de transport d'électrons.
